# EUROPÄISCHE PATENTSCHRIFT

(11) **EP 4 171 773 B1**
(45) Veröffentlichungstag und Bekanntmachung des Hinweises auf die Patenterteilung: **10.07.2024**
(21) Anmeldenummer: 21732904.4
(22) Anmeldetag: 17.06.2021
(51) Int. Cl.: B01D 3/32, C07C 51/44

(54) **VORRICHTUNG ZUR DURCHFÜHRUNG VON STOFFAUSTAUSCHPROZESSEN**
DEVICE FOR CARRYING OUT MATERIAL EXCHANGE PROCESSES
DISPOSITIF DE MISE EN OEUVRE DE PROCESSUS DE TRANSFERT DE MATIÈRE

(30) Priorität: 29.06.2020 EP 20182884
(43) Veröffentlichungstag der Anmeldung: 03.05.2023
(73) Patentinhaber: BASF SE, 67056 Ludwigshafen am Rhein (DE)
(72) Erfinder: METZEN, Bernd, 67056 Ludwigshafen (DE); KUNKELMANN, Christian, 67056 Ludwigshafen (DE); LANG, Ortmund, 67056 Ludwigshafen (DE); KRAMP, Marvin, 67056 Ludwigshafen (DE); HECHLER, Claus, 67056 Ludwigshafen (DE)
(74) Vertreter: BASF IP Association
(86) Internationale Anmeldenummer: PCT/EP2021/066379
(87) Internationale Veröffentlichungsnummer: WO 2022/002608

(56) Entgegenhaltungen:
- EP-A1- 2 380 645
- WO-A1-2017/005565
- US-A- 4 019 964
- US-A1- 2004 182 693
- US-A1- 2016 158 667

## Beschreibung

Die Erfindung geht aus von einer Vorrichtung zur Durchführung von Stoffaustauschprozessen, umfassend eine Kolonne mit mindestens zwei Stutzen zur Zufuhr einer dampfförmigen Phase, wobei die mindestens zwei Stutzen einen Höhenversatz aufweisen, der maximal dem Dreifachen des Stutzendurchmessers entspricht.

Kolonnen zur Durchführung von Stoffaustauschprozessen mit mindestens zwei Stutzen zur Zufuhr einer dampfförmigen Phase finden insbesondere Einsatz, wenn große Durchsätze realisiert werden sollen und die Kolonne mit entsprechend großem Durchmesser gestaltet wird. Übliche Kolonnendurchmesser sind dabei größer als 2 m. Die Anzahl der Stutzen ist insbesondere abhängig von der Anzahl an erforderlichen Verdampfern, die eine begrenzte Baugröße und unterschiedliche Energieträger aufweisen können. Üblicherweise wird je Verdampfer ein Stutzen an der Kolonne vorgesehen. Stoffaustauschprozesse mit mindestens einer dampfförmigen Phase, die in Kolonnen durchgeführt werden, sind zum Beispiel Destillationen, Absorptionen oder Gaswäschen.

US 4,019,964 beschreibt ein Verfahren zur Regelung einer Wärmezufuhr zu Verdampfern einer Destillationskolonne sowie die Verwendung von zwei Verdampfern. Auch CN 203861950 erwähnt die Verwendung von zwei Verdampfern. Weiter offenbaren Dokumente US2004/182693, US2016/158667, WO2017/005565 und EP2380645 Kolonnen mit mehreren Stutzen.

Derzeit werden Stutzen zur Zufuhr einer dampfförmigen Phase, die im Wesentlichen auf gleicher Höhe an der Kolonne angebracht sind, üblicherweise gleichverteilt über den Umfang der Kolonne angeordnet. Bei zwei Stutzen bedeutet dies zum Beispiel, dass die Stutzen einen Winkel von 180° zueinander aufweisen.

Zur Intensivierung des Wärme- und Stoffaustauschs zwischen den unterschiedlichen Phasen werden üblicherweise Kolonnen eingesetzt, die trennwirksame Einbauten enthalten. Im Allgemeinen umfassen derartige Prozesse mindestens eine dampfförmige und mindestens eine flüssige Phase. Unter trennwirksamen Einbauten werden Einbauten verstanden, an denen die mindestens eine dampfförmige Phase und die mindestens eine flüssige Phase miteinander in Kontakt gebracht werden, so dass eine Grenzfläche vergrößert und ein Stoffaustausch zwischen der mindestens einen dampfförmigen Phase und der mindestens einen flüssigen Phase intensiviert wird. Als trennwirksame Einbauten werden zum Beispiel Böden, die auch als Stoffaustauschböden bezeichnet werden können, strukturierte Packungen oder Füllkörperschüttungen eingesetzt. Die Stutzen zur Zufuhr der dampfförmigen Phase befinden sich dabei üblicherweise unterhalb der trennwirksamen Einbauten. Bei einer Anordnung der Stutzen im Bereich des Sumpfes der Kolonne sind die Stutzen unterhalb sämtlicher in der Kolonne enthaltenen trennwirksamen Einbauten angeordnet, bei einer Anordnung als Seitenzulauf befinden sich die Stutzen zwischen zwei Sektionen, die auch als horizontale Teilbereiche der Kolonne bezeichnet werden können, mit trennwirksamen Einbauten.

Bei der thermodynamischen Auslegung einer Kolonne werden eine bestimmte Trennleistung und eine bestimmte Energiezufuhr festgelegt, um eine definierte Auftrennung eines Stoffgemischs zu erzielen. Diese Festlegung ist direkt mit einem bestimmten Verhältnis von dampfförmiger Phase zu flüssiger Phase in der Kolonne gekoppelt. Lokale Abweichungen des Verhältnisses von dampfförmiger Phase zu flüssiger Phase, basierend auf einer Ungleichverteilung der dampfförmigen Phase oder der flüssigen Phase bezogen auf die Querschnittsfläche der Kolonne, müssen durch eine gegenüber der Auslegung erhöhte Energiezufuhr kompensiert werden, um die definierte Auftrennung eines Stoffgemischs zu erreichen.

Da in einem Stoffaustauschprozess in einer Kolonne also eine gleichmäßige Verteilung der dampfförmigen Phase in den trennwirksamen Einbauten erforderlich ist, um einen möglichst intensiven Stoffaustausch zu erhalten, muss eine gleichmäßige Verteilung der Dampfströmung im Bereich der Stutzen, durch die die dampfförmige Phase zugeführt wird, realisiert werden. Es hat sich jedoch gezeigt, dass eine gleichmäßige Verteilung der Stutzen über den Umfang der Kolonne, insbesondere bei zwei Stutzen, keine optimale Verteilung der Dampfströmung ergibt, da die einzelnen Ströme aufeinanderprallen und so zu einer Verungleichmäßigung führen, also eine weniger gleichmäßige Strömung bilden.

Aufgabe der vorliegenden Erfindung ist es daher, eine Vorrichtung zur Durchführung von Stoffaustauschprozessen bereitzustellen, bei der eine gleichmäßigere Verteilung der dampfförmigen Phase unterhalb der trennwirksamen Einbauten im Bereich der Stutzen, durch die die dampfförmige Phase zugeführt wird, erhalten wird. Weiterhin ist es Aufgabe der vorliegenden Erfindung eine Verwendung der Vorrichtung in einem Verfahren bereitzustellen, wodurch Energie eingespart werden kann.

Die Aufgabe wird einerseits gelöst mit einer Vorrichtung zur Durchführung von Stoffaustauschprozessen, umfassend eine Kolonne und mindestens zwei, insbesondere genau zwei, Stutzen zur Zufuhr einer dampfförmigen Phase, wobei in der Kolonne trennwirksame Einbauten aufgenommen sind und sich ein Kolonnenabschnitt von den mindestens zwei Stutzen zu den trennwirksamen Einbauten erstreckt, in dem eine Bedeckung einer Querschnittsfläche der Kolonne weniger als 25%, bevorzugt weniger als 20%, mehr bevorzugt weniger als 10% und weiter bevorzugt weniger als 5% beträgt, bezogen auf die gesamte Querschnittsfläche der Kolonne, und wobei die mindestens zwei Stutzen einen Höhenversatz aufweisen, der maximal dem Dreifachen eines Stutzendurchmessers entspricht, und die mindestens zwei Stutzen einen Winkel α von 60° bis 150°, bevorzugt von 80° bis 130°, mehr bevorzugt von 90° bis 120°, zum Beispiel von 95° bis 115°, zueinander aufweisen und eine Asymmetrie zueinander aufweisen. Der Kolonnenabschnitt erstreckt sich insbesondere von einer obersten Kante der mindestens zwei Stutzen zu einem, insbesondere untersten, Eintritt in die trennwirksamen Einbauten. Der Kolonnenabschnitt weist bevorzugt eine Abschnittshöhe in einem Bereich von 0 bis zum Dreifachen des Stutzendurchmessers auf, mehr bevorzugt in einem Bereich von einem Faktor 0,5 bis 1,5 des Stutzendurchmessers. Weisen die mindestens zwei Stutzen verschiedene Stutzendurchmesser auf, so beziehen sich diese Angaben auf den größten Stutzendurchmesser. Die Abschnittshöhe ist insbesondere der kleinste Abstand zwischen den trennwirksamen Einbauten und einem der mindestens zwei Stutzen. Bevorzugt beträgt die Bedeckung aller Querschnittsflächen des Kolonnenabschnitts weniger als 25%, mehr bevorzugt weniger als 20%, mehr bevorzugt weniger als 10% und weiter bevorzugt weniger als 5%, bezogen auf die jeweilige gesamte Querschnittsfläche der Kolonne. Insbesondere ist die Querschnittsfläche der Kolonne zwischen den mindestens zwei Stutzen und den trennwirksamen Einbauten frei, was auch als unbedeckt bezeichnet werden kann. Bevorzugt ist der Kolonnenabschnitt unverbauter Raum und frei von trennwirksamen Einbauten und weiteren Einbauten, die jeweils eine Bedeckung der Querschnittsfläche darstellen können, so dass sich ungehindert eine räumlich gleichverteilte Strömung vor Eintritt in die trennwirksamen Einbauten einstellen kann, die in den mindestens zwei Stutzen ihren Ursprung hat. Einbauten, die zu einer Bedeckung von mehr als 25% und damit zu einer Behinderung der Strömung von den mindestens zwei Stutzen zu den trennwirksamen Einbauten führen könnten, sind beispielsweise horizontale Einbauten, wie mindestens ein Boden wie ein Sammelboden oder eine Platte, zum Beispiel eine Lochplatte, und/oder vertikale Einbauten wie mindestens ein Rohr, insbesondere mit oder ohne Haube zur Abdeckung, wie ein Kamin, der in der Regeln den Kontakt zwischen flüssiger und dampfförmiger Phase verhindert, beispielsweise auf einem Sammelboden. Weiterhin ist die Kolonne insbesondere eine Kolonne ohne Trennwand. Gegebenenfalls vorhandene Befestigungsvorrichtungen der trennwirksamen Einbauten wie beispielsweise Tragroste, die der Fixierung insbesondere der Packungen beziehungsweise Füllkörper in der Kolonne dienen oder Träger zur Stabilisierung der Böden sowie Schächte, insbesondere Ablaufschächte, von trennwirksamen Böden, werden dabei als Teil der trennwirksamen Einbauten betrachtet.

Der Begriff Asymmetrie ist im Rahmen der vorliegenden Erfindung als Bezeichnung für Ungleichheit zu verstehen, wobei zum Beispiel eine asymmetrische Anordnung der Stutzen zu ungleichen Umfangsteilen führt. Die Asymmetrie ist dadurch gegeben, dass die mindestens zwei Stutzen jeweils unterschiedliche Stutzendurchmesser aufweisen und bevorzugt zudem asymmetrisch über den Umfang der Kolonne verteilt sind. Die asymmetrische Verteilung über den Umfang kann auch dadurch beschrieben werden, dass die mindestens zwei Stutzen ungleich über den Umfang der Kolonne verteilt sind.

Bevorzugt unterscheiden sich die Stutzendurchmesser der mindestens zwei Stutzen um mindestens 10%, weiter bevorzugt um mindestens 20% und insbesondere um mindestens 25%, bezogen auf den kleinsten Stutzendurchmesser. Der Stutzendurchmesser bezeichnet insbesondere den mittleren Stutzendurchmesser des Stutzens am Eintritt in die Kolonne Bevorzugt unterscheidet sich der Winkel α um mindestens 10°, mehr bevorzugt mindestens 30°, weiter bevorzugt mindestens 60°, von einem weiteren Winkel β zwischen zwei der mindestens zwei Stutzen. Im Fall von genau zwei Stutzen unterscheidet sich der Winkel α bevorzugt um mindestens 120°, mehr bevorzugt um mindestens 180° von einem weiteren Winkel β zwischen den zwei Stutzen. Der Winkel α und der weitere Winkel β bezeichnen insbesondere Winkel zwischen zwei benachbarten Stutzen. Der Winkel α ist bevorzugt der kleinste Winkel zwischen zwei Stutzen.

Weiterhin kann die Asymmetrie darin bestehen oder dadurch gegeben sein, dass die mindestens zwei Stutzen mit jeweils unterschiedlichen mittleren Geschwindigkeiten durchströmt werden. Bevorzugt unterscheiden sich die mittleren Geschwindigkeiten in den mindestens zwei Stutzen um mindestens 10%, weiter bevorzugt um mindestens 30% und insbesondere um mindestens 45%, bezogen auf die kleinste Geschwindigkeit. Zur Bestimmung der mittleren Geschwindigkeit können zum Beispiel Durchflussmessungen, insbesondere bei rein dampfförmigen Zuläufen, durchgeführt werden. Bei Verdampfern kann zum Beispiel auch die eingesetzte Dampfmenge zur Beheizung des zu verdampfenden Mediums als proportionales Maß für die Dampfdurchflussmenge in den Stutzen bestimmt werden.

Bei einer Anzahl von mehr als zwei Stutzen können die Stutzen bevorzugt unterschiedliche Winkel zueinander aufweisen. Entsprechend sind die mehr als zwei Stutzen in diesem Fall asymmetrisch über den Umfang der Kolonne verteilt. Für die Bemessung des Höhenversatzes wird bevorzugt die Lage der Mittelpunkte der Querschnitte der jeweiligen Stutzen betrachtet.

Die mindestens zwei Stutzen ermöglichen es, eine größere Menge an dampfförmiger Phase zuzuführen, als dies mit nur einem Stutzen möglich ist. Insbesondere kann bei gleicher Menge die dampfförmige Phase mit einer geringeren Geschwindigkeit zugeführt werden, wodurch die Energie, mit der die zugeführten Ströme an dampfförmiger Phase aufeinanderprallen, reduziert wird und eine gleichmäßigere Strömungsverteilung erzielt werden kann.

Durch eine gleichmäßigere Strömungsverteilung kann Energie im Stoffaustauschprozess, in dem die Vorrichtung eingesetzt wird, eingespart werden, da lokale Inhomogenitäten bezüglich des Verhältnisses von flüssiger Phase zu dampfförmiger Phase verringert oder vermieden werden.

Als Maß für die gleichmäßige Strömungsverteilung wird die in axiale Richtung weisende Komponente der Geschwindigkeit der dampfförmigen Phase am Eintritt in die trennwirksamen Einbauten der Kolonne herangezogen. Hierzu wird die Differenz zwischen der Geschwindigkeit, die so groß ist, dass nur auf 5% der Querschnittsfläche die Geschwindigkeit größer ist, und der Geschwindigkeit, die so groß ist, dass nur auf 5% der Querschnittsfläche die Geschwindigkeit kleiner ist, gebildet. Je kleiner diese Differenz, bezogen auf die mittlere Geschwindigkeit, ist, umso gleichmäßiger ist die Strömungsverteilung.

Überraschenderweise hat sich gezeigt, dass, anders als bei einer gleichmäßigen beziehungsweise symmetrischen Verteilung der mindestens zwei Stutzen über den Umfang der Kolonne, mit einer Anordnung der Stutzen mit einem Winkel im Bereich von 60° bis 150° zueinander und einer Asymmetrie der mindestens zwei Stutzen eine gleichmäßigere Strömungsverteilung erzielt werden kann. Insbesondere ist es durch diese Anordnung der mindestens zwei Stutzen nicht erforderlich, die in die Kolonne eintretende dampfförmige Phase unterhalb der trennwirksamen Einbauten durch weitere Maßnahmen wie zusätzliche Einbauten zu führen und oder zu kanalisieren, da bereits durch das gezielte Aufeinandertreffen der Ströme aus den mindestens zwei Stutzen, also in dem entsprechenden Winkel, ein bezüglich der Strömungsgeschwindigkeit homogenisiertes Strömungsfeld in die trennwirksamen Einbauten eintritt. Bei einer Anzahl von mehr als zwei Stutzen ist die Anordnung dabei bevorzugt so, dass die mehr als zwei Stutzen jeweils unterschiedliche Winkel zueinander aufweisen, um eine gleichmäßigere Strömungsverteilung der dampfförmigen Phase zu erhalten.

Stoffaustauschprozesse, die mit der erfindungsgemäßen Vorrichtung durchgeführt werden können, sind alle Stoffaustauschprozesse, bei denen mindestens eine flüssige und mindestens eine dampfförmige Phase beteiligt sind. Als dampfförmige Phase wird im Rahmen der vorliegenden Erfindung auch eine siedende Phase verstanden, das heißt, eine Phase, die sowohl einen flüssigen als auch einen dampfförmigen Anteil enthält. Übliche Stoffaustauschprozesse sind zum Beispiel Absorptionen, Destillationen, Rektifikationen, Extraktionen oder Gaswäschen.

Um die Bauhöhe der Kolonne so gering wie möglich zu halten, sind die mindestens zwei Stutzen in einer bevorzugten Ausführungsform in gleicher Höhe an der Kolonne angeordnet. In gleicher Höhe bedeutet hier, dass die Mittelpunkte der mindestens zwei Stutzen auf gleicher Höhe sind, wobei eine Abweichung im Rahmen der Fertigungstoleranzen möglich ist. Die Anordnung der mindestens zwei Stutzen auf gleicher Höhe hat den weiteren Vorteil, dass die Bedingungen, bei denen die dampfförmige Phase über die mindestens zwei Stutzen zugeführt wird, insbesondere Druck und Temperatur für alle Stutzen gleich sind, so dass durch die Höhe der mindestens zwei Stutzen keine Abweichung der physikalischen Eigenschaften der dampfförmigen Phase auftritt.

Die mindestens zwei Stutzen, durch die die dampfförmige Phase zugeführt wird, sind üblicherweise am Sumpf der Kolonne oder als Seitenzulauf an der Kolonne angeordnet. Sind die mindestens zwei Stutzen als Seitenzulauf angeordnet, bezeichnet der Begriff trennwirksame Einbauten im Rahmen der Erfindung die trennwirksamen Einbauten, die als nächstes oberhalb der mindestens zwei Stutzen angeordnet sind. Bei einem zweiphasigen Stoffaustauschprozess strömt üblicherweise die flüssige Phase von oben nach unten und die dampfförmige Phase von unten nach oben. Daher wird am Kopf der Kolonne flüssige Phase zugeführt und die dampfförmige Phase am Sumpf und/oder über Seitenzuläufe. Wenn die dampfförmige Phase eine siedende Phase ist, wird diese besonders bevorzugt über einen Seitenzulauf zugeführt. In der Kolonne erfolgt dann eine Phasentrennung und der dampfförmige Teil der siedenden Phase strömt nach oben und der flüssige Teil nach unten. Selbstverständlich ist es auch möglich, die siedende Phase im Bereich des Sumpfes der Kolonne zuzuführen. In diesem Fall strömt der gasförmige Teil nach oben und der flüssige Teil sammelt sich im Sumpf und wird über einen Sumpfabzug aus der Kolonne entnommen.

Um den Stoffaustausch in der Kolonne zu intensivieren, sind trennwirksame Einbauten, bevorzugt ausgewählt aus der Gruppe bestehend aus Böden wie Dual-Flow-Böden, Kaskadenböden, Ripple-Tray-Böden und Querstromböden und strukturierten und unstrukturierten Packungen und Kombinationen davon, in der Kolonne aufgenommen. Durch die trennwirksamen Einbauten erfolgt eine ständige Umlenkung und Neuverteilung der flüssigen Phase und der dampfförmigen Phase und zudem wird die Phasengrenze hierdurch deutlich vergrößert, so dass eine sehr viel größere Phasengrenzfläche, an der der Stoffaustausch erfolgt, erhalten wird.

Bei Böden wird üblicherweise unterschieden zwischen Böden mit einer Zwangsführung der flüssigen Phase, wie Querstromböden, und Böden ohne eine Zwangsführung. In einer Ausführungsform umfassen die trennwirksamen Einbauten bevorzugt Böden ohne Zwangsführung wie Dual-Flow-Böden, Ripple-Tray-Böden und/oder Kaskadenböden. In einer anderen Ausführungsform umfassen die trennwirksamen Einbauten Querstromböden. Mehr bevorzugt umfassen die trennwirksamen Einbauten ausschließlich Böden, insbesondere Böden ohne Zwangsführung wie Dual-Flow-Böden, Ripple-Tray-Böden und/oder Kaskadenböden. Böden mit einer Zwangsführung weisen mindestens einen Ablaufschacht für Flüssigkeit auf, durch den die flüssige Phase auf den darunter liegenden Boden abläuft. Der Ablaufschacht wirkt hierdurch gleichzeitig als Zulauf für den Boden, der unter dem Ablaufschacht positioniert ist. Die Ablaufschächte übereinander liegender Böden können an gegenüberliegenden Positionen angeordnet sein.

Unter Dual-Flow-Böden, Ripple-Tray-Böden und Kaskadenböden werden Böden ohne eine Zwangsführung der flüssigen Phase verstanden. Ripple-Tray-Böden werden auch als Wellsiebböden bezeichnet. Die flüssige Phase läuft durch Öffnungen im Boden auf den darunter liegenden Boden ab und die dampfförmige Phase strömt von unten durch die Öffnungen auf den Boden. Öffnungen können dabei nur von der dampfförmigen Phase oder nur von der flüssigen Phase oder von der dampfförmigen Phase und der flüssigen Phase oder von keiner Phase durchströmt werden. Damit die flüssige Phase vom Boden ablaufen kann, weisen die Öffnungen in einem Dual-Flow-Boden keinen Kamin auf. Stoffaustauschkolonnen mit Dual-Flow-Böden sind beispielsweise in der WO-A 03/043712 oder in der WO-A 2004/063138 beschrieben.

Eingesetzte Packungen können strukturierte Packungen oder unstrukturierte Packungen sein. Unstrukturierte Packungen sind zum Beispiel Füllkörperschüttungen, wobei als Füllkörper alle üblichen, dem Fachmann bekannten Füllkörper zum Einsatz kommen können. Geeignete Füllkörper sind zum Beispiel Ringe, Gitter, Wendel und/oder Sattelkörper wie Raschig-, IMTP^{®}- oder Pall-Ringe, Berl- oder Intalox-Sättel oder Geflechte. Als strukturierte Packungen können Packungen in unterschiedlicher geometrischer Ausführung eingesetzt werden, beispielsweise Metallblechpackungen oder Metallgewebepackungen. In einer Ausführungsform umfassen die trennwirksamen Einbauten bevorzugt strukturierte Packungen und/oder Füllkörperschüttungen und umfassen mehr bevorzugt ausschließlich strukturierte Packungen und/oder Füllkörperschüttungen.

Die trennwirksamen Einbauten sind bevorzugt aus einem Material aufgebaut, das Metall, Keramik, Glas, Carbon, Graphit, Kunststoff oder Mischungen daraus umfasst. Weiter bevorzugt bestehen die trennwirksamen Einbauten aus Metall, Keramik, Glas, Carbon, Graphit, Kunststoff oder Mischungen daraus.

Die Wirksamkeit der trennwirksamen Einbauten innerhalb der Vorrichtung zur Durchführung von Stoffaustauschprozessen sollte bevorzugt mindestens 2 theoretischen Trennstufen, zum Beispiel 2 bis 40 theoretischen Trennstufen entsprechen. Besonders bevorzugt ist eine Trennleistung von 10 bis 30 theoretischen Trennstufen.

Wenn die mindestens zwei Stutzen als Seitenzulauf an der Kolonne angeordnet sind, werden diese üblicherweise zwischen zwei Böden oder - wenn die trennwirksamen Einbauten strukturierte oder unstrukturierte Packungen umfassen - zwischen zwei Segmenten mit trennwirksamen Einbauten angeordnet, so dass die dampfförmige Phase unterhalb eines Bodens oder einer Packung zugeführt wird und gleichmäßig von unten durch den Boden strömen oder in die Packung eintreten kann.

Sowohl als Seitenzulauf als auch bei Anordnung der mindestens zwei Stutzen im Bereich des Sumpfes, sind die mindestens zwei Stutzen bevorzugt in einem Abstand unterhalb der trennwirksamen Einbauten angeordnet, der der Abschnittshöhe entspricht. Die Stutzendurchmesser der mindestens zwei Stutzen unterscheiden sich bevorzugt um nicht mehr als einen Faktor von 3, mehr bevorzugt um einen Faktor von 1,5, bezogen auf den kleinsten Stutzendurchmesser. Die mindestens zwei Stutzen können den gleichen Stutzendurchmesser aufweisen. Ferner beträgt ein Verhältnis des Stutzendurchmessers zum Kolonnendurchmesser bevorzugt maximal 0,8. Weisen die mindestens zwei Stutzen verschiedene Stutzendurchmesser auf, so beziehen sich diese Angaben auf den größten Stutzendurchmesser.

In einer bevorzugten Ausführungsform ist der in der Kolonne durchgeführte Stoffaustauschprozess eine Destillation oder Rektifikation und die Kolonne eine Destillationskolonne oder Rektifikationskolonne. In diesem Fall sind über die mindestens zwei Stutzen zur Zufuhr der dampfförmigen Phase vorzugsweise Verdampfer an die Kolonne angebunden. Mehr bevorzugt ist pro Stutzen jeweils ein, insbesondere genau ein, Verdampfer an die Kolonne angebunden. In die Verdampfer wird Flüssigkeit eingeleitet, die Flüssigkeit im Verdampfer teilweise oder vorzugsweise vollständig verdampft und der Dampf als dampfförmige Phase über die Stutzen in die Kolonne eingeleitet. Die Flüssigkeit kann dabei den Verdampfern von außen zugeführt werden oder es wird flüssige Phase aus der Kolonne entnommen und in den Verdampfer eingeleitet. Hierbei besteht weiterhin die Möglichkeit, dass ein Teil der im Verdampfer verdampften Flüssigkeit von außen zugeführt wird und ein Teil der Kolonne entnommen wird. Flüssige Phase, die der Kolonne entnommen und den Verdampfern zugeführt wird, kann zum Beispiel am Sumpf der Kolonne entnommen werden. Wenn der Verdampfer als Zwischenverdampfer eingesetzt wird, ist es bevorzugt, wenn die flüssige Phase über einen Seitenabzug, zum Beispiel von einem Boden, der Kolonne abgezogen und dem Verdampfer zugeführt wird.

Eine weitere Möglichkeit ist, dass ein Verdampfer dazu eingesetzt wird, von außen zugeführte Flüssigkeit zu verdampfen und ein zweiter Verdampfer zur Verdampfung von flüssiger Phase, die aus der Kolonne entnommen wird. Bevorzugt ist es jedoch, in diesem Fall die von außen zugegebene Flüssigkeit und die der Kolonne entnommene flüssige Phase jedem Verdampfer zuzuführen, wobei die Flüssigkeiten entweder vor Zugabe in den Verdampfer gemischt werden oder über getrennte Leitungen in den Verdampfer eingeleitet und im Verdampfer gemischt werden.

Als Verdampfer kann dabei jeder dem Fachmann bekannte Verdampfertyp eingesetzt werden, der für den Stoffaustauschprozess, insbesondere die Destillation oder Rektifikation, geeignet ist. Als Verdampfer eignen sich zum Beispiel Rohrbündelverdampfer und Plattenverdampfer. Die Verdampfer können als Fallfilmverdampfer, Zwangsumlaufverdampfer, Zwangsumlaufentspannungsverdampfer, Wendelrohrverdampfer, Kettletypeverdampfer oder Naturumlaufverdampfer ausgeführt sein.

In einer bevorzugten Ausführungsform der Erfindung münden die mindestens zwei Stutzen radial in die Kolonne. Dies ist insbesondere dahingehend zu verstehen, dass Verlängerungen der Mittelachsen der mindestens zwei Stutzen die Mittelachse der Kolonne schneiden. Die Stutzen können mit der Kolonnenwand abschließen oder in die Kolonne hineinragen, bevorzugt schließen die Stutzen mit der Kolonnenwand ab. Durch eine Anordnung der mindestens zwei Stutzen derart, dass diese unterschiedliche Winkel α beziehungsweise β zueinander aufweisen, und/oder unterschiedliche Stutzendurchmesser beziehungsweise Strömungsgeschwindigkeiten aufweisen, prallen die zugeführten Dampfströme nicht symmetrisch aufeinander. Hierdurch entsteht eine gleichmäßigere Dampfströmung in der Kolonne und es bildet sich keine Dampfkonzentration in der Kolonnenmitte.

Alternativ zur radialen Anordnung ist es auch möglich, dass die mindestens zwei Stutzen in einem Mündungswinkel zur radialen Richtung in die Kolonne münden.

Die mindestens zwei Stutzen, durch die die dampfförmige Phase zugegeben wird, können jede beliebige Querschnittsform aufweisen. Bevorzugt ist es jedoch, wenn die mindestens zwei Stutzen einen kreisförmigen oder elliptischen Querschnitt haben. Die Querschnittsfläche der mindestens zwei Stutzen ist abhängig von der Menge der zuzuführenden dampfförmigen Phase. Möglich ist es, dass die Querschnittsfläche der auf im Wesentlichen gleicher Höhe angeordneten mindestens zwei Stutzen im Wesentlichen gleich groß ist. Im Wesentlichen gleich groß bedeutet dabei, dass die Querschnittsflächen sich in ihrer Größe aufgrund von Fertigungstoleranzen unterscheiden können. Auch ist die Form der Querschnittsflächen der im Wesentlichen auf gleicher Höhe angeordneten mindestens zwei Stutzen vorzugsweise gleich.

Alternativ ist es selbstverständlich auch möglich, dass Querschnittsfläche und/oder Querschnittsform der auf im Wesentlichen gleicher Höhe angeordneten mindestens zwei Stutzen unterschiedlich sind. Mit unterschiedlichen Querschnittsformen können lokale konstruktive Bedingungen berücksichtigt werden. Unterschiedliche Querschnittsflächen dienen der Anpassung an Verdampfer mit unterschiedlichen Energiequellen, die einen unterschiedlichen Energiegehalt aufweisen wie zum Beispiel bei einer Wärmeintegration. Bei der Wärmeintegration wird ein Teil der benötigten Wärme von einer weiteren Wärmequelle direkt aus dem Prozess bezogen. Da die verfügbare Wärmemenge aus der Integration und die verbleibende benötigte Wärmemenge stark unterschiedlich sein können, ergeben sich hier unterschiedliche Querschnittsflächen der jeweiligen Stutzen. Ferner kann die Querschnittsfläche in Abhängigkeit von der Größe des zuzuführenden Dampfstroms gewählt werden.

Um die beste Ausrichtung der mindestens zwei Stutzen zueinander zu finden, ist es vorteilhaft, wenn die Anordnung der mindestens zwei Stutzen durch eine mathematische Simulation berechnet wird. Ein hierzu geeignetes Verfahren umfasst folgende Schritte:
(a) Vorgabe der Position und Ausrichtung der mindestens zwei Stutzen an der Kolonne;
(b) Berechnen der Gasströmung in der Kolonne mit einer Strömungssimulation;
(c) Wiederholen der Schritte (a) und (b) mit unterschiedlichen Positionen und Ausrichtungen der mindestens zwei Stutzen und
(d) Auswahl der Position und Ausrichtung der mindestens zwei Stutzen bei der Strömung, die den gleichmäßigsten Strömungsverlauf zeigt.

Zur Strömungssimulation können alle dem Fachmann bekannten Simulationsprogramme eingesetzt werden. Hierbei eignen sich insbesondere numerische Simulationen auf Basis von finiten Elementen oder finiten Volumen, bevorzugt auf Basis von finiten Volumen. Ein geeignetes Simulationsprogramm ist zum Beispiel das kommerziell erhältliche ANSYS Fluent^{®}, das auf Basis finiter Volumen arbeitet. Mit Hilfe der Strömungssimulation kann die Strömung in der Kolonne grafisch dargestellt werden und anhand des Ergebnisses können die Positionen der mindestens zwei Stutzen zueinander optimiert werden. Bei unterschiedlicher Querschnittsform und/oder Querschnittsfläche der mindestens zwei Stutzen lassen sich durch die Strömungssimulation auch die optimale Größe und Form der Querschnittsfläche der mindestens zwei Stutzen bestimmen, die zur gleichmäßigsten Strömungsverteilung in der Kolonne führen.

Weiterhin wird die Verwendung der Vorrichtung zur Durchführung von Stoffaustauschprozessen zur Herstellung, insbesondere zur kontinuierlichen Herstellung, von Isocyanaten, Styrol oder eines Acrylsäurealkylesters, insbesondere eines Acrylsäurebutylesters, oder in Crackern, insbesondere zum Splitten von C₃-Kohlenwasserstoffen, vorgeschlagen. Die Verwendung der Vorrichtung zur Durchführung von Stoffaustauschprozessen zur kontinuierlichen Herstellung des Acrylsäurealkylesters ist insbesondere von Vorteil, da das Verfahren durch einen hohen Energiebedarf gekennzeichnet ist.

Die Vorrichtung zur Durchführung von Stoffaustauschprozessen wird vorteilhaft eingesetzt in einem Verfahren zur kontinuierlichen Herstellung eines Acrylsäurebutylesters H₂C=CH-C(=O)OR, mit R = n-Butyl oder iso-Butyl.

Acrylsäurealkylester können aus 3-Hydroxypropionsäure hergestellt werden, wie beispielsweise in der WO 2019/034577 beschrieben. Alternativ kann Acrylsäure zur Herstellung des Acrylsäurebutylesters eingesetzt werden.

3-Hydroxypropionsäure kann zunächst in einem ersten Schritt mit einem Alkohol verestert und dann in einem nachfolgenden Schritt der resultierende 3-Hydroxypropionsäureester zum entsprechenden Acrylsäurealkylester dehydratisiert werden. Alternativ kann 3-Hydroxypropionsäure auch zunächst in einem ersten Schritt dehydratisiert werden und dann in einem nachfolgenden Schritt die resultierende Acrylsäure mit einem Alkohol verestert werden.

Bevorzugt wird in dem Verfahren zur kontinuierlichen Herstellung des Acrylsäurebutylesters (H₂C=CH-C(=O)OR, mit R = n-Butyl oder iso-Butyl) wässrige 3-Hydroxypropionsäure in Gegenwart des Alkohols n-Butanol umsetzt.

Weiter bevorzugt wird die erfindungsgemäße Vorrichtung zur Durchführung von Stoffaustauschprozessen als Rektifikationskolonne in dem Verfahren zur kontinuierlichen Herstellung des Acrylsäurealkylesters (H₂C=CH-C(=O)OR, mit R = n-Butyl oder iso-Butyl) eingesetzt, wobei wässrige 3-Hydroxypropionsäure unter dehydratisierenden und veresternden Bedingungen in Gegenwart des entsprechenden Butanols (R-OH) in einem Reaktor mit der Rektifikationskolonne umgesetzt wird und gebildeter Acrylsäurebutylester, unumgesetztes Butanol sowie eingesetztes und gebildetes Wasser als ternäres Azeotrop über Kopf abdestilliert wird und, nach Trennung in eine jeweils flüssige wässrige und organische Phase, die wässrige und die organische Phase jeweils zumindest teilweise ausgeschleust werden und die organische Phase, enthaltend den Acrylsäurebutylester und das Butanol, destillativ aufgetrennt wird.

Durch den Einsatz von 3-Hydroxypropionsäure kann ein nahezu acetatfreier Acrylsäurebutylester hergestellt werden. Unter ,Acetat' ist hier n-Butyl- bzw. iso-Butylacetat [H₃C-C(=O)-OR] zu verstehen.

Bevorzugt handelt es sich bei der eingesetzten 3-Hydroxypropionsäure um biobasierte 3-Hydroxypropionsäure. Unter "biobasierter 3-Hydroxypropionsäure" ist eine solche zu verstehen, die ausgehend von nachwachsenden Rohstoffen hergestellt wurde. Weiter bevorzugt ist, dass die biobasierte 3-Hydroxypropionsäure durch Fermentation, insbesondere aus Glukose, Xylose, Arabinose, Sukrose, Fruktose, Cellulose, Glukose-Oligomeren und/oder Glycerin durch Fermentation, besonders mit anschließender Reinigung, hergestellt wurde. Zum Beispiel ist aus WO 2012/074818 A2 die Herstellung von biobasierter 3-Hydroxypropionsäure, die auch als Bio-3-Hydroxypropionsäure oder Bio-HPS bezeichnet wird, aus Zuckern wie Glukose durch Fermentation und anschließender Reinigung bekannt.

Die so erzeugte wässrige Bio-3-Hydroxypropionsäure enthält beispielsweise neben Wasser im Wesentlichen folgende Bestandteile:
35 bis 70 Gew.-% 3-Hydroxypropionsäure,
0 bis 20 Gew.-% oligomere 3-Hydroxypropionsäure,
0 bis 10 Gew.-% Acrylsäure,
0 bis 1 Gew.-% oligomere Acrylsäure,
0,01 bis 0,1 Gew.-% Glykolsäure,
0,01 bis 0,1 Gew.-% 2-Hydroxypropionsäure,
0,005 bis 0,05 Gew.-% Ameisensäure,
0 bis 0,15 Gew.-%, insbesondere 0,0 bis 0,05 Gew.-%, z.B. 0,005 bis 0,10 Gew.-%, Essigsäure,
0,005 bis 0,05 Gew.-% Bernsteinsäure,
0,005 bis 0,05 Gew.-% Fumarsäure,
0,0001 bis 0,01 Gew.-% Formaldehyd,
0,0001 bis 0,01 Gew.-% Acetaldehyd,
0,0001 bis 0,01 Gew.-% Methanol und
0,0001 bis 0,01 Gew.-% Ethanol.

Bevorzugt beträgt das molare Einsatzverhältnis von Butanol zu 3-Hydroxypropionsäure mindestens 1 und liegt weiterhin bevorzugt unterhalb von 5. Besonders vorteilhaft ist ein molares Einsatzverhältnis von Butanol zu 3-Hydroxypropionsäure im Bereich von 1 : 1 bis 3 : 1. Ganz besonders bevorzugt ist ein molares Einsatzverhältnis im Bereich von 1,1 : 1 bis 1,8 : 1.

Bevorzugt liegen die dehydratisierenden und gleichzeitig veresternden Bedingungen vor, wobei eine katalytisch wirksame Menge einer Säure zugegen ist. Vorteilhaft beträgt der Gehalt an katalytisch wirksamer Säure im Reaktor, bezogen auf das darin enthaltene Reaktionsgemisch, 0,1 Gew.-% bis 20 Gew.-%, weiter bevorzugt 5 Gew.-% bis 15 Gew.-%, insbesondere 7 Gew.-% bis 10 Gew.-%. Bevorzugte Säuren sind anorganische Säuren, wie Schwefelsäure, Phosphorsäure, sowie organische Sulfonsäure. Unter den organischen Sulfonsäuren sind Methansulfonsäure, Benzolsulfonsäure, Dodecylbenzolsulfonsäure und/oder p-Toluolsulfonsäure bevorzugt. Auch kann ein Gemisch aus jeweils mindestens einer organischen Sulfonsäure und anorganischer Säure, zum Beispiel Schwefelsäure, eingesetzt werden. Besonders bevorzugt ist es, als Veresterungs- und Dehydratisierungskatalysator(en) Schwefelsäure und/oder organische Sulfonsäure(n) einzusetzen.

Die Umsetzung der Reaktanden, also der Edukte 3-Hydroxypropionsäure und Butanol, im Reaktor erfolgt bevorzugt bei einer Temperatur im Bereich von 80°C bis 170°C, mehr bevorzugt im Bereich von 100°C bis 155°C, weiter bevorzugt im Bereich von 120°C bis 140°C. Die Verweilzeit der Reaktanden, also der Edukte 3-Hydroxypropionsäure und Butanol, im Reaktor beträgt bevorzugt 1 Stunde bis 20 Stunden, weiter bevorzugt 2 Stunden bis 8 Stunden. Unter der Verweilzeit versteht man die Zeit, welche eine Sumpfabzugsmenge des Reaktors in dem Flüssigkeitsvolumen des Reaktors verweilt.

Im einfachsten Fall ist die Rektifikationskolonne direkt auf den Reaktor aufgesetzt, wobei in der Regel die aus dem Reaktor aufsteigenden Brüden, also die dampfförmige Phase, zu den in die Rektifikationskolonne geführten Rücklaufmengen, also der flüssigen Phase, im Gegenstrom geführt werden. Die direkt aufgesetzte Rektifikationskolonne bietet den Vorteil, die im Reaktor entstehenden Brüden unmittelbar ohne zusätzliche Rohrleitungen in die Rektifikationskolonne und die in der Rektifikationskolonne ablaufende flüssige Phase direkt in den Reaktor zu befördern.

Alternativ ist eine getrennte Anordnung von Reaktor und Rektifikationskolonne möglich, mit entsprechenden Rohrleitungen zur Zuführung der dampfförmigen Phase in die Rektifikationskolonne sowie zum Ablauf der durch die Rektifikationskolonne strömenden flüssigen Phase in den Reaktor. Auch solch eine Ausführungsform mit indirekt aufgesetzter Kolonne ist vom Begriff ,Reaktor mit Rektifikationskolonne' umfasst.

Der Druck am Kopf der Rektifikationskolonne liegt bevorzugt im Bereich von 0,2 bar bis 5,0 bar, weiter bevorzugt im Bereich von 0,3 bar bis 3,0 bar, insbesondere im Bereich von 0,5 bar bis 1,2 bar.

Bevorzugt erfolgt die Trennung in eine wässrige und eine organische Phase mittels eines Phasenscheiders. In einem solchen Apparat können zwei nicht miteinander homogen mischbare Flüssigkeiten durch deren Dichteunterschied getrennt werden. Zumindest ein Teil der anfallenden wässrigen Phase, die neben Wasser auch noch Butanol und in Spuren gegebenenfalls weitere Komponenten enthält, wird bevorzugt ausgeschleust. Besonders bevorzugt werden 10 Gew.-% bis 80 Gew.-%, weiter besonders 20 Gew.-% bis 70 Gew.-%, der anfallenden wässrigen Phase ausgeschleust. Der Rest wird jeweils bevorzugt in die Rektifikationskolonne zurückgeführt. Bevorzugt wird ein Teil der anfallenden organischen Phase ebenfalls, bevorzugt in die Rektifikationskolonne, zurückgeführt. Bevorzugt werden 0 Gew.-% bis 80 Gew.-%, zum Beispiel 1 Gew.-% bis 75 Gew.-%, mehr bevorzugt 5 Gew.-% bis 50 Gew.-%, der organischen Phase, bevorzugt in die Rektifikationskolonne, zurückgeführt. Der andere Teil wird bevorzugt ausgeschleust und einer destillativen Auftrennung zugeführt.

Die destillative Auftrennung der ausgeschleusten organischen Phase enthaltend den Acrylsäurebutylester und das Butanol erfolgt bevorzugt derart, dass in einer nachgeschalteten Rektifikationskolonne das Butanol über Kopf abgetrennt wird, wie zum Beispiel in EP 765 859 A1 beschrieben. Die erfindungsgemäße Vorrichtung zur Durchführung von Stoffaustauschprozessen kann zur destillativen Auftrennung der ausgeschleusten organischen Phase verwendet werden. Die Kolonne, die von der erfindungsgemäßen Vorrichtung zur Durchführung von Stoffaustauschprozessen umfasst ist, kann als die nachgeschaltete Rektifikationskolonne verwendet werden. Vorzugsweise führt man das so abgetrennte Butanol in den Reaktor zurück. Zweckmäßigerweise erfolgt die Rückführung kontinuierlich, mit oder ohne Zwischenbehälter.

Bevorzugt erfolgt die destillative Auftrennung der organischen Phase, enthaltend den Acrylsäurebutylester und das Butanol, derart, dass in einer zusätzlichen Rektifikationskolonne das Butanol abdestilliert und aus der resultierenden Sumpfflüssigkeit in einer weiteren zusätzlichen Rektifikationskolonne der Acrylsäurebutylester abdestilliert wird.

Die resultierende Sumpfflüssigkeit der zusätzlichen Rektifikationskolonne besteht im Wesentlichen aus dem Acrylsäurebutylester sowie geringen Mengen an Schwersiedern und einem eingesetzten Stabilisator, der auch als Prozessstabilisator bezeichnet werden kann und zum Beispiel Phenothiazin (PTZ) enthält oder daraus besteht.

In einer weiteren nachgeschalteten Rektifikationskolonne wird der Acrylsäurebutylester üblicherweise über Kopf abgetrennt. Die erfindungsgemäße Vorrichtung zur Durchführung von Stoffaustauschprozessen kann zur Abtrennung des Acrylsäurebutylesters verwendet werden. Die Kolonne, die von der erfindungsgemäßen Vorrichtung zur Durchführung von Stoffaustauschprozessen umfasst ist, kann als die weitere nachgeschaltete Rektifikationskolonne verwendet werden. Bei der Kondensation wird bevorzugt ein Stabilisator, insbesondere ein Lagerstabilisator wie p-Methoxyphenol (MeHQ) zugegeben. Die schwerer siedende Nebenprodukte enthaltende Sumpfflüssigkeit dieser weiteren nachgeschalteten Rektifikationskolonne wird zweckmäßigerweise bevorzugt in den Reaktor zurückgeführt, vorzugsweise kontinuierlich, mit oder ohne Zwischenbehälter.

Eine besondere Ausführungsform besteht darin, dass der Acrylsäurebutylester aus der nachgeschalteten Rektifikationskolonne zur Rückgewinnung von Butanol durch einen Seitenabzug nach Abscheidung eventuell mitgerissener Flüssigkeitströpfchen entnommen und zum Reinester kondensiert wird. Bei der Kondensation wird diesem ein Stabilisator, insbesondere ein Lagerstabilisator wie MeHQ zugegeben. In diesem Ausführungsfall wird der Sumpfaustrag der nachgeschalteten Rektifikationskolonne, der im Wesentlichen aus Acrylsäurebutylester besteht, vorzugsweise zurück in den Reaktor geführt. Das nach der Auftrennung erhaltene Butanol wird besonders vorteilhaft zumindest teilweise zur Umsetzung in den Reaktor zurückgeführt. Bevorzugt werden 5 Gew.-% bis 100 Gew.-%, weiter bevorzugt 80 Gew.-% bis 100 Gew.-%, des Butanols zurückgeführt.

Mit dem Verfahren zur kontinuierlichen Herstellung des Acrylsäurebutylesters H₂C=CH-C(=O)OR, mit R = n-Butyl, kann Acrylsäure-n-butylester mit insbesondere einer Reinheit von ≥ 99,0 Gew.-%, weiter besonders ≥99,5 Gew.-%, und einem Gehalt an n-Butylacetat von ≤ 1000 ppm, weiter besonders von ≤ 100 ppm, hergestellt werden. Insbesondere beträgt der Gehalt an Acrylsäure < 100 ppm, z.B. 5 bis 80 ppm.

Mit dem Verfahren zur kontinuierlichen Herstellung des Acrylsäurebutylesters H₂C=CH-C(=O)OR, mit R = iso-Butyl, kann Acrylsäure-iso-butylester mit insbesondere einer Reinheit von ≥ 99,0 Gew.-%, weiter besonders ≥ 99,5 Gew.-%, und einem Gehalt an iso-Butylacetat von ≤ 1000 ppm, weiter besonders von ≤ 100 ppm, hergestellt werden. Insbesondere beträgt der Gehalt an Acrylsäure < 100 ppm, z.B. 5 bis 80 ppm.

Im Verfahren zur kontinuierlichen Herstellung des Acrylsäurebutylesters H₂C=CH-C(=O)OR, mit R = n-Butyl oder iso-Butyl, wird der gebildete Acrylsäurebutylester bevorzugt durch geeignete Polymerisationsinhibitoren stabilisiert, um eine ungewünschte Polymerisation zu vermeiden. Das Verfahren wird bevorzugt in Gegenwart wirksamer Mengen eines Stabilisators oder mehrerer Stabilisatoren durchgeführt. Als Stabilisatoren eignen sich prinzipiell alle Polymerisationsinhibitoren, die zur Stabilisierung von Acrylsäure und Acrylsäureestern in z.B. DE 10 2005 053 982 A1 und DE 102 58 329 A1 empfohlen werden. Geeignete Stabilisatoren können beispielsweise N-Oxide (Nitroxyl- oder N-Oxyl-Radikale, also Verbindungen, die wenigstens eine N-O-Gruppe aufweisen), wie z.B. 4-Hydroxy-2,2,6,6-tetramethylpiperidin-N-oxyl (4HT) oder 4-Oxo-2,2,6,6-tetramethylpiperidin-N-oxyl, Phenole und Naphthole, wie p-Methoxyphenol, p-Aminophenol, p-Nitrosophenol, 2-tert.-Butylphenol, 4-tert.-Butylphenol, 2,4-Di-tert.-butylphenol, 2-Methyl-4-tert.-butylphenol, 2,6-tert.-Butyl-4-methylphenol oder 4-tert.-Butyl-2,6-dimethylphenol, Chinone, wie z.B. Hydrochinon oder Hydrochinonmonomethylether, aromatische Amine, wie z.B. N,N-Diphenylamin, Phenylendiamine, wie z.B. N,N'-Dialkyl-p-phenylendiamin, wobei die Alkylreste gleich oder verschieden sein können und jeweils unabhängig voneinander 1 bis 4 Kohlenstoffatome aufweisen und geradkettig oder verzweigt sein können, wie z.B. N,N'-Dimethyl-p-phenylendiamin oder N,N'-Diethyl-p-phenylendiamin, Hydroxylamine, wie z.B. N,N-Diethylhydroxylamin, Imine, wie z.B. Methylethylimin oder Methylenviolett, Sulfonamide, wie z.B. N-Methyl-4-toluolsulfonamid oder N-tert.-Butyl-4-toluolsulfonamid, Oxime, wie Aldoxime, Ketoxime oder Amidoxime, wie z.B. Diethylketoxim, Methylethylketoxim oder Salicyladoxim, phosphorhaltige Verbindungen, wie z.B. Triphenylphosphin, Triphenylphosphit oder Triethylphosphit, schwefelhaltige Verbindungen wie z.B. Diphenylsulfid oder Phenothiazin, Metallsalze, wie z.B. Cer(III)acetat oder Cer(III)ethylhexanoat, aber auch verschiedene Kupfersalze etwa Cu(II)dialkyldithiocarbamate wie z.B. das Cu(II)dibutyldithiocarbamat und auch Cu(II)oxinat (Oxin = 4-Hydroxychinolin), zudem Mangansalze wie z.B das Mn(II)diacetat oder Gemische davon, sein. Bevorzugt erfolgt die Stabilisierung mit Phenothiazin (PTZ), MeHQ, Hydrochinon, Hydrochinonmonomethylether, 4-Hydroxy-2,2,6,6-tetramethylpiperidin-N-oxyl, 4-Oxo-2,2,6,6-tetramethylpiperidin-N-oxyl, 2,6-tert.-Butyl-4-methylphenol oder Gemischen davon. Ganz besonders bevorzugt wird Phenothiazin (PTZ) und/oder MeHQ und/oder 4-Hydroxy-2,2,6,6-tetramethylpiperidin-N-oxyl (4HT) als Polymerisationsinhibitor verwendet.

Auch wenn die Inhibitoren als Reinsubstanz zugegeben werden können, ist es von Vorteil, den Inhibitor gelöst in einem Lösungsmittel als einfach und reproduzierbar zu dosierende Lösung zuzugeben, wobei grundsätzlich auch Inhibitormischungen in einer einzelnen Lösung möglich sind. Bevorzugt wird eine im Acrylatsyntheseverfahren bzw. dem Stoffgemisch in der Rektifikationskolonne bereits vorhandene Flüssigkeit als Lösungsmittel verwendet. Besonders bevorzugt für die Wahl als Lösungsmittel ist das Acrylatprodukt (also der Acrylsäurebutylester) selbst, Wasser oder einer der Syntheseeinsatzstoffe für das Acrylat (z.B. das Butanol).

Insbesondere am unteren Ende der Rektifikationskolonne wird die in der Rektifikationskolonne ablaufende Flüssigkeit, also die flüssige Phase, bevorzugt zumindest teilweise der Rektifikationskolonne entnommen, zumindest teilweise in mindestens einem Verdampfer, bevorzugt in mindestens zwei, zum Beispiel genau zwei, Verdampfern verdampft und zumindest teilweise über die mindestens zwei Stutzen in die Rektifikationskolonne zurückgeführt.

Das Verfahren zur kontinuierlichen Herstellung des Acrylsäurebutylesters H₂C=CH-C(=O)OR, mit R = n-Butyl oder iso-Butyl, wird vorteilhaft mit besonderen Maßnahmen zur Kontrolle bestimmter Parameter durchgeführt. Diese Prozesskontrolle erfolgt bevorzugt wie folgt:
Für die Produktion von spezifikationsgerechtem Acrylsäurebutylester, also einem Produkt mit hoher Reinheit von insbesondere mehr als 99 Gew.-%, ist die Abtrennung von Acrylsäure vom Acrylsäurebutylester in der Rektifikationskolonne von entscheidender Bedeutung. Dabei hat sich als vorteilhaft erwiesen, ein definiertes Verhältnis zwischen dem organischen und dem wässrigen Rücklauf einzustellen. Dieses Rücklaufverhältnis der Ströme liegt bevorzugt im Bereich von 0,1 bis 1,0.

Weiterhin wird das für den Umsatz ausschlaggebende Reaktionsvolumen im Sumpf der Rektifikationskolonne oder dem eigenständigem Reaktor bei indirekt aufgesetzter Rektifikationskolonne bevorzugt konstant oder nahezu konstant gehalten. Unter nahezu konstant wird im Rahmen der vorliegenden Erfindung verstanden, dass eine Abweichung von bis zu einschließlich +/- 10 Vol.% vorliegt. Dies lässt sich zu einem dadurch erreichen, dass bei konstantem oder nahezu konstantem Flüssigkeitsstand im Reaktor ein konstanter oder nahezu konstanter Flüssigkeitsstrom aus dem Reaktionsvolumen ausgeschleust wird. Außerdem steht bevorzugt die Sumpfabzugsmenge in einem bestimmten Verhältnis zum Zulauf, bevorzugt in einem Verhältnis der Sumpfabzugsmenge zu Zulauf im Bereich von 0,01 bis 0,30.

Eine zweite Maßnahme ist eine Qualitätskontrolle bezüglich des Acrylsäuregehaltes im organischen Destillat. Da das Flüssigkeitsvolumen in dem Reaktionsraum stark auf die wässrige Rücklaufmenge reagiert, wird der Flüssigkeitsstand im Reaktor bevorzugt mittels der Rücklaufmenge, die der Rückführmenge entspricht, der wässrigen Phase geregelt.

Der wässrige Rücklauf gewährleistet, dass die Schwersieder n- bzw. iso-Butylacrylat und das entsprechende Butanol aufgrund der Bildung eines Leichtsiederazeotrops abdestilliert werden können. Der organische Rücklauf gewährleistet, dass die Konzentration der im Reaktor entstandenen Acrylsäure unter der Konzentration von insbesondere 100 ppm bleibt.

Durch die Steuerung der Menge des organischen Rücklaufs können mehrere Effekte kombiniert werden wie Reinigen durch Destillieren, Erhöhung der Verweilzeit im Reaktionsraum, Erhöhung der Konzentration an Butanol im Reaktionsraum. Diese Regelstrategie führt zu einem besonders stabilen Betrieb im Reaktor und in der Rektifikationskolonne.

Durch das verbesserte Regelkonzept kann eine nochmals höhere Ausbeute an Acrylsäurebutylester bei niedrigerem Energieverbrauch und nochmals verbesserter Qualität, insbesondere verbesserter Reinheit, erzeugt werden.

In einer bevorzugten Ausführungsform ist in der Rektifikationskolonne mindestens ein erster Stabilisator zugegen, der sich in wirksamen Anteilen sowohl in der wässrigen als auch in der organischen Phase löst. Besonders wird ein solcher Stabilisator, wie insbesondere 4HT, oberhalb der obersten theoretischen Trennstufe der Rektifikationskolonne zugegeben. Dadurch ist die gesamte Rektifikationskolonne mit dem Stabilisator stabilisiert.

Weiterhin wird bevorzugt mindestens ein weiterer Stabilisator, der sich in wirksamen Anteilen sowohl in der wässrigen als auch in der organischen Phase löst, in den Phasenscheider, der das Kondensat auffängt, und/oder in die Leitung eines Quenchkreislaufs und/oder am Kopf des Kondensators zugegeben. Dieser weitere Stabilisator gleicht bevorzugt dem ersten Stabilisator und ist insbesondere 4HT.

Der bevorzugt eingerichtete Quenchkreislauf (d.h. der flüssige Rückführstrom eines Teils des Kondensats, z.B. 10 bis 50 Gewichtshundertstel des Kondensats, in den Kondensator) hat die Funktion, dass die natürlicherweise stabilisatorfreien Brüden beim Kondensieren im Kondensator besonders ausreichend stabilisiert sind.

In der jeweiligen Phase in Lösung befindliche wirksame Stabilisatormengen liegen insgesamt besonders bei ≥ 10 Gew.-ppm, z.B. im Bereich von 10 bis 1000 Gew.-ppm.

Wenn sich ein eingesetzter Stabilisator in der jeweiligen flüssigen Phase nicht vollständig löst, ist er entsprechend suspendiert zugegen. Bei Vorhandensein eines Stabilisators als Suspension in der oder den flüssigen Phasen kann dieser ä priori kaum oder nicht wirksame partikuläre Stabilisatoranteil durch seine Wirkung als Stabilisator-Depot Vorteile bieten, da z.B. im Falle des chemischen Abbaus von gelöstem Stabilisator, wodurch dessen Wirksamkeit verschlechtert wird, weiterer, dann frisch wirksamer Stabilisator aus dem suspendierten Anteil zusätzlich in Lösung geht, was bei geeignet innigem Kontakt der flüssigen Phasen auch phasen-übergreifend eintreten kann und über die Größenverteilung der Partikel beeinflussbar ist.

Die Stabilisatoren können jeweils insbesondere als Lösung in einem geeigneten Lösungsmittel, besonders wie oben aufgeführt, z.B. dem im Verfahren verwendeten Alkohol, also Butanol, Wasser, dem entsprechenden Acrylsäurebutylester, z.B. jeweils als 1 bis 5 gew.-%ige Lösung, eingesetzt werden.

In den Reaktor wird vorteilhaft ein zweiter Stabilisator, insbesondere PTZ, der für höhere Temperaturen geeignet ist, und ein dritter Stabilisator, insbesondere MeHQ, der aufgrund seines höheren Dampfdruckes auch noch den Übergangsbereich zwischen Reaktionsraum und Kolonnenunterteil stabilisiert, zugegeben. Der zweite und dritte Stabilisator können jeweils insbesondere als Lösung in einem geeigneten Lösungsmittel, besonders wie oben aufgeführt, z.B. dem im Verfahren entsprechend gebildeten Acrylsäurebutylester oder in den verwendeten Edukten 3-Hydroxypropionsäure oder Butanol eingesetzt werden.

Vorteilhaft wird zusätzlich ein Sauerstoff-haltiges Gas zur Polymerisationsinhibierung eingesetzt. Besonders geeignet sind hierzu Luft/Stickstoff-Gemische, z.B. mit einem Sauerstoffgehalt von 4 Vol.-% bis 9 Vol.-%. Wird ein Sauerstoff-haltiges Gas zur Polymerisationsinhibierung eingesetzt, so wird dies vorzugsweise am unteren Ende des Verdampfers oder am unteren Ende des Reaktors zugeführt.

Das Anfahren des Verfahrens zur kontinuierlichen Herstellung des Acrylsäurebutylesters (H₂C=CH-C(=O)OR, mit R = n-Butyl oder iso-Butyl) umfassend die Umsetzung im Reaktor und die Destillation in der Rektifikationskolonne kann problembehaftet sein, da insbesondere Änderungen der Rücklaufmengen der verschiedenen Ströme sich stark unterschiedlich auf das Gesamtsystem auswirken. Änderungen der Menge des wässrigen Rücklaufs wirken sich relativ schnell auf die gebildete Dampfmenge und Änderungen der Menge des organischen Rücklaufs wirken sich relativ langsam auf die Acrylsäurekonzentration im Kopf der Kolonne aus. Aber beide Rücklaufmengen sind nicht unabhängig voneinander. Wenn die genauen Rücklaufmengen nicht gut aufeinander abgestimmt sind, dann kann die Verdampfung zum Erliegen kommen, oder die Rektifikationskolonne kommt aufgrund zu hoher Dampfmenge zum Fluten. Es ist dann sehr schwierig, das System wieder in den normalen Betriebszustand zu überführen. Vorteilhaft wird daher zum Anfahren der Reaktor zunächst mit entsprechender Menge eines entsprechenden Reaktionsgemisches enthaltend den Acrylsäurebutylester, insbesondere Sumpfprodukt aus einer vorhergehenden Produktionskampagne, oder des entsprechenden Acrylsäurebutylesters befüllt. Dann werden der Sumpf auf Betriebstemperatur, also Umsetzungstemperatur, erhitzt und die Zuläufe aus 3-Hydroxypropionsäure, Butanol und Katalysator in Betrieb genommen.

Durch die besondere Anfahrstrategie und/oder das besondere Stabilisierungskonzept können weiter erhöhte Ausbeuten und/oder Produktreinheiten erzielt werden.

Alle Druckangaben beziehen sich auf den Absolutdruck. Alle ppm-Angaben beziehen sich auf das Gewicht.

Ausführungsbeispiele der Erfindung sind in den Figuren illustriert und werden in der nachfolgenden Beschreibung näher erläutert.

Es zeigen:
- Figuren 1 bis 5: Querschnittansichten einer Kolonne mit verschiedenen Stutzenanordnungen,
- Figur 6: eine dreidimensionale Darstellung einer Kolonne mit zwei Stutzen,
- Figur 7: eine dreidimensionale Darstellung einer Kolonne mit zwei Stutzen unterschiedlichen Durchmessers, wie in der Erfindung,
- Figur 8: eine Längsschnittansicht der Kolonne mit Stutzen,
- Figur 9: ein Histogramm mit Darstellung der relativen Geschwindigkeit am Eintritt in trennwirksame Einbauten in Form einer Packung,
- Figur 10: Häufigkeitsfunktionen der relativen Geschwindigkeit am Eintritt in trennwirksame Einbauten in Form einer Packung für unterschiedliche Stutzenanordnungen,
- Figur 11: Häufigkeitsfunktionen der relativen Geschwindigkeit am Eintritt in trennwirksame Einbauten in Form von Böden für unterschiedliche Stutzenanordnungen,
- Figur 12: Häufigkeitsfunktionen der relativen Geschwindigkeit am Eintritt in trennwirksame Einbauten in Form von Böden für unterschiedliche Stutzenanordnungen und ungleiche Strömungsgeschwindigkeiten in den Stutzen,
- Figur 13: Häufigkeitsfunktionen der relativen Geschwindigkeit am Eintritt in trennwirksame Einbauten in Form von Böden für unterschiedliche Stutzenanordnungen und ungleiche Stutzendurchmesser,
- Figur 14: Häufigkeitsfunktionen der relativen Geschwindigkeiten für einen und zwei Stutzen und
- Figur 15: eine schematische Darstellung einer Vorrichtung zur Durchführung von Stoffaustauschprozessen.

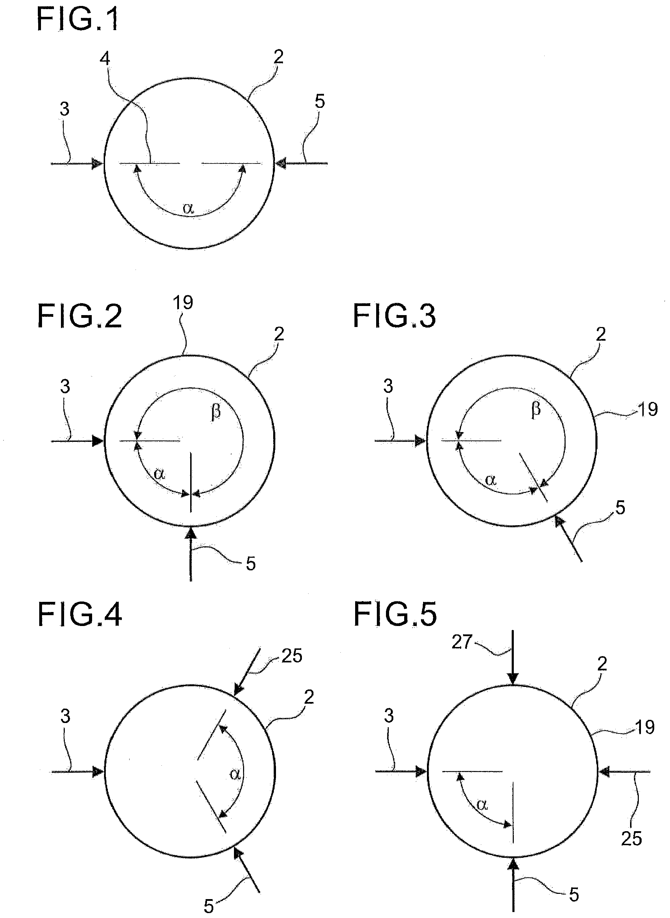

Figuren 1 bis 5 zeigen Querschnittansichten einer Kolonne 2 mit verschiedenen Anordnungen von Stutzen 3, 5.

Gemäß Figur 1 sind entsprechend dem Stand der Technik ein erster Stutzen 3 und ein zweiter Stutzen 5 in einem Winkel α von 180° an der Kolonne 2 angeordnet. Ferner sind die Stutzen 3, 5 in einer radialen Richtung 4 an der Kolonne 2 ausgerichtet.

Auch die Kolonnen 2, die in den Figuren 2 und 3 dargestellt sind, besitzen genau zwei Stutzen 3, 5. Hier beträgt der Winkel α zwischen den beiden Stutzen 3, 5 weniger als 180°. Gemäß Figur 2 beträgt der Winkel α 90° und gemäß Figur 3 beträgt der Winkel α 120°. Ein weiterer Winkel β zwischen den beiden Stutzen 3, 5 beträgt gemäß Figur 2 270° und gemäß Figur 3 240°. Entsprechend weisen die beiden Stutzen 3, 5 aufgrund ihrer Ungleichverteilung um den Umfang 19 der Kolonne 2 eine Ungleichheit zueinander auf, da unterschiedlich lange Umfangsabschnitte zwischen den Stutzen 3, 5 gebildet werden.

An der Kolonne 2 in Figur 4 sind drei Stutzen 3, 5, 25 zur Zufuhr einer dampfförmigen Phase angeordnet. Der Winkel α zwischen den Stutzen 3, 5, 25 beträgt jeweils 120° und die Stutzen 3, 5 sind gleichverteilt über den Umfang 19 angeordnet. Es handelt sich um eine Ausführungsform gemäß dem Stand der Technik, sofern gleiche Stutzendurchmesser 6, 17 und gleiche Geschwindigkeiten in den Stutzen 3, 5, 25 vorliegen. Bei mindestens zwei unterschiedlichen Stutzendurchmessern 6, 17 und/oder mindestens zwei unterschiedlichen Geschwindigkeiten der wie dargestellt angeordneten Stutzen 3, 5, 25 liegt eine erfindungsgemäße Ausführungsform vor.

Figur 5 zeigt eine Kolonne 2 mit vier Stutzen 3, 5, 25, 27. Diese sind gleichmäßig über den Umfang 19 der Kolonne 2 verteilt und der Winkel α zwischen den Stutzen 3, 5, 25, 27 beträgt jeweils 90°. Es handelt sich ebenfalls um eine Ausführungsform gemäß dem Stand der Technik, sofern gleiche Stutzendurchmesser 6, 17 und gleiche Geschwindigkeiten in den Stutzen 3, 5, 25, 27 vorliegen. Bei mindestens zwei unterschiedlichen Stutzendurchmessern 6, 17 und/oder mindestens zwei unterschiedlichen Geschwindigkeiten der wie dargestellt angeordneten Stutzen 3, 5, 25, 27 liegt eine erfindungsgemäße Ausführungsform vor.

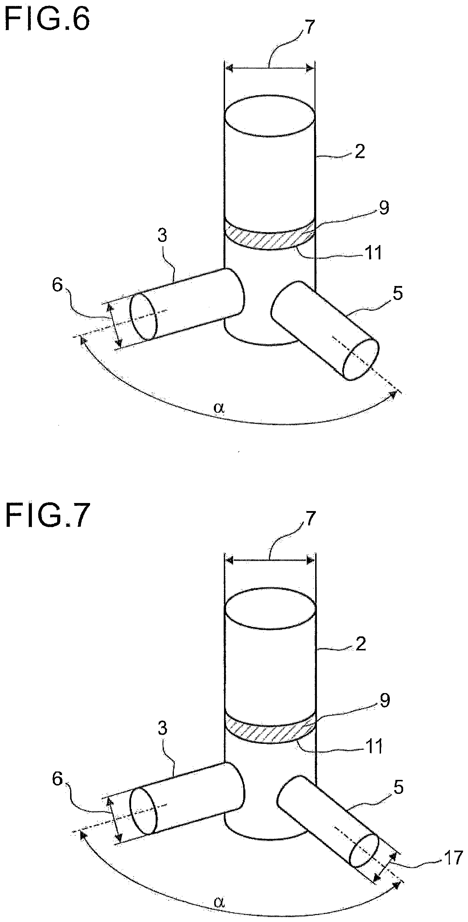

Figur 6 zeigt eine dreidimensionale Darstellung einer Kolonne 2 mit zwei Stutzen 3, 5. Zur besseren Übersicht ist nur der Bereich der Kolonne 2 gezeigt, in dem die Stutzen 3, 5 angeordnet sind.

An der Kolonne 2 sind zur Zufuhr einer dampfförmigen Phase zwei Stutzen 3, 5 angeordnet. Die Stutzen 3, 5 weisen zueinander einen Winkel α auf, der im Bereich von 60° bis 150° liegt. In der hier dargestellten Ausführungsform beträgt der Winkel α 90°. Bei der Anordnung der Stutzen 3, 5 sind insbesondere keine Stutzen 3, 5 unmittelbar gegenüber angeordnet. Weiterhin ist es vorteilhaft, wenn mehr als zwei Stutzen 3, 5 vorgesehen sind, dass die Winkel zwischen den Stutzen 3, 5 unterschiedlich sind. Hierdurch wird ein direktes Aufprallen der über die Stutzen 3, 5 zugeführten dampfförmigen Phasen vermieden und auf diese Weise eine gleichmäßigere Strömungsverteilung erzielt.

Oberhalb der Stutzen 3, 5 zur Zufuhr der dampfförmigen Phase befinden sich trennwirksamen Einbauten 9 in der Kolonne 2, in Form einer Packung mit einem Eintritt 11.

Figur 7 zeigt eine dreidimensionale Darstellung einer Kolonne 2 mit zwei Stutzen 3, 5, die im Wesentlichen der Kolonne 2 gemäß Figur 6 entspricht mit dem Unterschied, dass hier Stutzen 3, 5 sich unterscheidende Stutzendurchmesser 6, 17 aufweisen. Der erste Stutzen 3 besitzt einen Stutzendurchmesser 6, der größer ist als ein weiterer Stutzendurchmesser 17 des zweiten Stutzens 5.

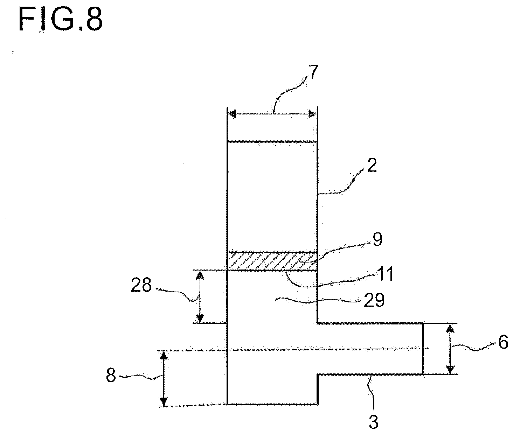

Figur 8 zeigt eine Längsschnittansicht der Kolonne 2 gemäß Figur 6, die einen Kolonnenabschnitt 29 und Stutzen 3, 5 aufweist. Der Kolonnenabschnitt 29 besitzt eine freie Querschnittsfläche und hat eine Abschnittshöhe 28. Die Stutzen 3, 5 haben den Stutzendurchmesser 6 und sind jeweils in einer Höhe 8 an der Kolonne 2 angeordnet, die einen Kolonnendurchmesser 7 aufweist.

### Beispiele und Vergleichsbeispiele

### Beispiel 1

Es wurde die Verteilung der relativen Geschwindigkeit am Eintritt 11 in eine Packung 9 einer Kolonne 2 ermittelt. Der Berechnung liegt eine Anordnung von zwei auf gleicher Höhe angeordneten Stutzen 3, 5 in einem Winkel α von 120° zueinander zugrunde.

Für die hier dargestellte Berechnung der Dampfströmung wurde als trennwirksame Einbauten 9 eine Packung mit einer Höhe von 1 m und einem Druckverlust von 1 mbar angesetzt. Der Kolonnendurchmesser 7 der Kolonne 2 wurde für die Berechnung mit 3200 mm und der Stutzendurchmesser 6 der beiden Stutzen 3, 5 mit 1000 mm angenommen. Für die Berechnung der Geschwindigkeiten wurden ein Druck in der Kolonne 2 von 5,5 bar, eine Gasdichte von 16,6 kg/m³, eine Gasviskosität von 1,3·10⁻⁵ Pa.s, eine Geschwindigkeit in den Stutzen 3, 5 von 1,07 m/s mit einem F-Faktor von 4,34 und eine Geschwindigkeit in der Kolonne 2 von 0,21 m/s mit einem F-Faktor von 0,85 als Randbedingungen vorgegeben. Der F-Faktor bezeichnet dabei die Dampfbelastung in der Kolonne 2 und ist das Produkt aus der mittleren Geschwindigkeit der dampfförmigen Phase in m/s multipliziert mit der Wurzel aus der Gasdichte in kg/m³.

Im Inneren der Kolonne 2 stellt sich ein System aus mehreren hier nicht dargestellten Wirbelstrukturen ein, in denen sich hier ebenfalls nicht dargestellte Stromlinien nach oben in Richtung zu den trennwirksamen Einbauten 9, also der Packung, bewegen.

Die vertikale Geschwindigkeitskomponente am Eintritt 11 der trennwirksamen Einbauten 9 ist ein Maß für die Fehlverteilung, die sich in der Kolonne 2 einstellt.

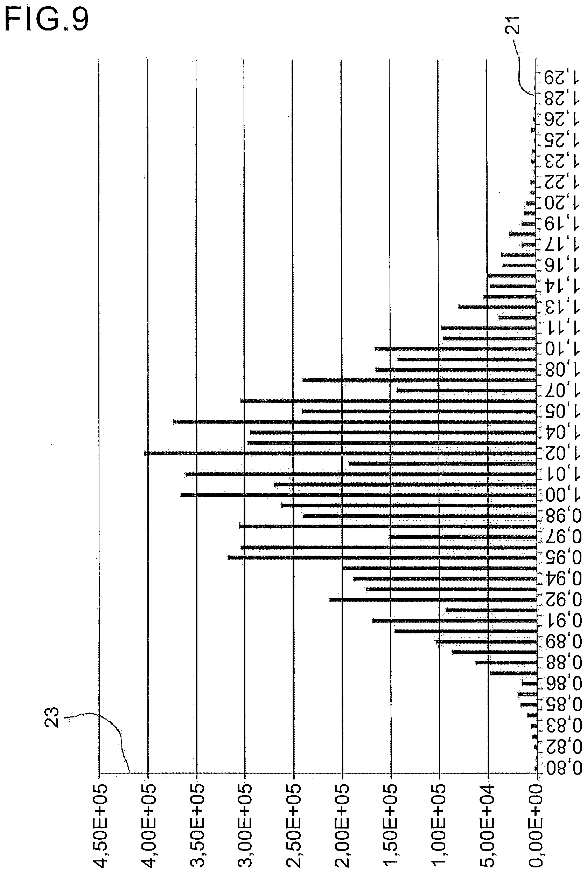

Um die Fehlverteilung als Maß für die Strömungsgleichmäßigkeit geeignet heranziehen zu können, ist es sinnvoll, zunächst die berechneten vertikalen Geschwindigkeiten am Eintritt 11 der Kolonne 2 in einem Histogramm darzustellen. Ein solches Histogramm zeigt beispielhaft Figur 9.

Zur Erzeugung des Histogramms ist es zum Beispiel möglich, zunächst die mit einem geeigneten Simulationsprogramm für Strömungsberechnungen berechneten vertikalen Geschwindigkeiten am Eintritt 11 in die trennwirksamen Einbauten 9 graphisch mit einer Grauskala darzustellen und aus den Graustufen das Histogramm zu erzeugen. Das Histogramm zeigt dabei für jede Geschwindigkeit den Anteil der Querschnittsfläche, in dem diese Geschwindigkeit auftritt. Hierbei ist die Geschwindigkeit auf der Abszisse 21 und die Querschnittsfläche auf der Ordinate 23 aufgetragen.

### Beispiel 2

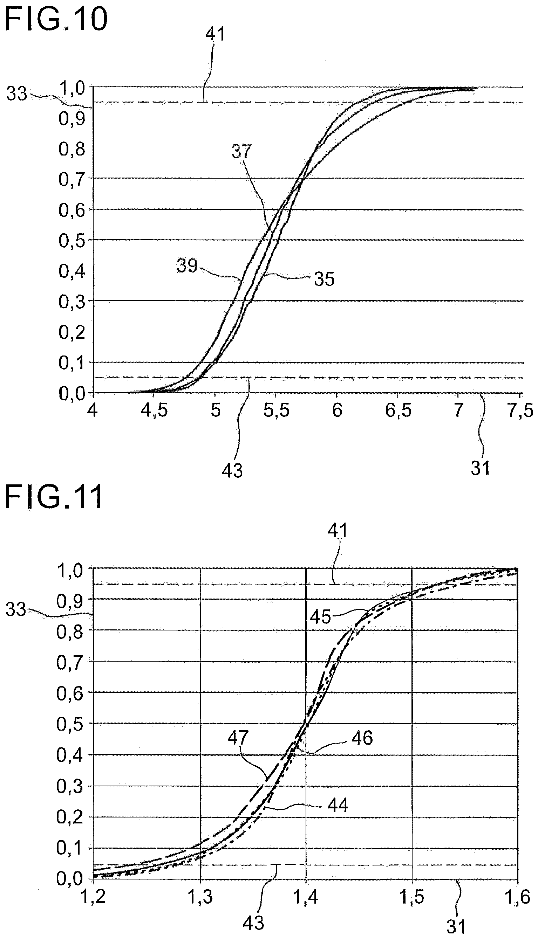

Aus Histogrammdaten, wie in Figur 9 dargestellt, wurde eine kumulierte Häufigkeitsfunktion gebildet, wie sie in den Figuren 10 bis 14 jeweils für unterschiedliche Stutzenanordnungen, Stutzenausführungen und Betriebsweisen gezeigt ist. Als Maß für die Ungleichverteilung wird die Differenz zwischen der Geschwindigkeit, die so groß ist, dass nur auf 5% der Querschnittsfläche die Geschwindigkeit größer ist, und der Geschwindigkeit, die so groß ist, dass nur auf 5% der Querschnittsfläche die Geschwindigkeit kleiner ist, gebildet. Je kleiner diese Differenz ist, umso gleichmäßiger ist die Strömungsverteilung.

Figur 10 zeigt Häufigkeitsfunktionen der relativen Geschwindigkeit am Eintritt 11 in die trennwirksamen Einbauten 9, also die Packung, für unterschiedliche Anordnungen der Stutzen 3, 5. Hierbei ist die Geschwindigkeit auf der Abszisse 31 und der kumulierte Flächenanteil von 0 (gar kein Anteil) bis 1 (die gesamte Fläche) auf der Ordinate 33 aufgetragen.

In einer ersten Anordnung beträgt der Winkel α zwischen den Stutzen 3, 5 90°. Die zugehörige erste Kurve der Häufigkeitsfunktion ist mit Bezugszeichen 35 bezeichnet. Eine zweite Kurve 37 zeigt die Häufigkeitsfunktion für eine Anordnung der Stutzen 3, 5 mit einem Winkel α von 120° und eine dritte Kurve 39 die Häufigkeitsfunktion für eine Anordnung der Stutzen 3, 5 mit einem Winkel α von 180°.

Im Unterschied zu dem Histogramm in Figur 9 wurden die Geschwindigkeiten in Figur 10 für eine Kolonne 2 mit einem Kolonnendurchmesser 7 von 6400 mm berechnet. Als weitere Randbedingungen wurden Stutzendurchmesser 6, 17 von jeweils 3000 mm, ein Druck von 0,025 bar, eine Gasdichte von 0,118 kg/m³, eine Gasviskosität von 7,8·10⁻⁶ Pas, eine Geschwindigkeit in den Stutzen 3, 5 von jeweils 11,7 m/s mit einem F-Faktor von 4 und eine Geschwindigkeit in der Kolonne 2 von 5,46 m/s mit einem F-Faktor von 1,87 vorgegeben.

Der Schnittpunkt der Kurven 35, 37, 39 mit einem kumulierten Flächenanteil 41 von 95% bezeichnet die Geschwindigkeit, die so groß ist, dass nur auf 5% der Querschnittsfläche die Geschwindigkeit größer ist und der Schnittpunkt der Kurven 35, 37, 39 mit dem kumulierten Flächenanteil 43 von 5% bezeichnet die Geschwindigkeit, die so groß ist, dass nur auf 5% der Querschnittsfläche die Geschwindigkeit kleiner ist. Die Differenz kann dann auf einfache Weise aus den Graphen bestimmt werden. Wenn alle Kurven 35, 37, 39 wie hier dargestellt in einem Diagramm gezeigt sind, kann die Ungleichverteilung unmittelbar abgelesen werden. Je größer der Abstand zwischen den Schnittpunkten jeweils einer Kurve 35, 37, 39 mit der Gerade 41 beziehungsweise 43 ist, umso größer ist die Ungleichverteilung. Bei einer Anordnung von 2 Stutzen 3, 5, ergibt sich damit, dass die größte Ungleichverteilung bei einem Winkel α der Stutzen von 180° vorliegt, so dass ein kleinerer Winkel α gewählt werden sollte. Der Unterschied der Ungleichverteilung für eine Anordnung der Stutzen bei 90° oder 120° ist im Vergleich zur Ungleichverteilung bei 180° so viel geringer, dass der exakte Winkel zum Beispiel den Gegebenheiten der Rohrführung um die Kolonne angepasst werden kann.

### Beispiel 3

In Figur 11 werden Häufigkeitsverteilungen gezeigt für eine Kolonne 2 mit einem Kolonnendurchmesser 7 von 2900 mm und einem Stutzendurchmesser 6, 17 der Stutzen 3, 5 von jeweils 900 mm. Als trennwirksamen Einbauten 9 in der Kolonne 2 wurden Böden mit einem Druckverlust von 3 mbar am Eintritt 11 in die trennwirksamen Einbauten 9, also den untersten Boden, angenommen. Der Berechnung lag eine Anordnung von zwei auf gleicher Höhe angeordneten Stutzen 3, 5 in einem Winkel α von jeweils 60°, 90°,120° oder 180° zueinander zugrunde.

Für die Berechnung der Geschwindigkeiten wurden ein Druck in der Kolonne 2 von 1,2 bar, eine Gasdichte von 1,63 kg/m³, eine Gasviskosität von 1,2·10⁻⁵ Pa·s, eine Geschwindigkeit in den Stutzen 3, 5 von jeweils 7,4 m/s mit einem F-Faktor von 9,4 und eine Geschwindigkeit in der Kolonne 2 von 1,82 m/s mit einem F-Faktor von 1,43 als Randbedingungen vorgegeben.

Im Inneren der Kolonne 2 stellt sich ein System aus mehreren hier nicht dargestellten Wirbelstrukturen ein, in denen sich hier ebenfalls nicht dargestellte Stromlinien nach oben in Richtung der Böden bewegen. Die vertikale Geschwindigkeitskomponente am Eintritt 11 in den untersten Boden ist ein Maß für die Fehlverteilung, die sich in der Kolonne 2 einstellt.

Aus den hier nicht dargestellten Histogrammdaten analog zu Figur 9 wurden kumulierte Häufigkeitsfunktionen jeweils für die unterschiedlichen Stutzenanordnungen gebildet, die in Figur 11 gezeigt sind.

Als Maß für die Ungleichverteilung wird die Differenz zwischen der Geschwindigkeit, die so groß ist, dass nur auf 5% der Querschnittsfläche die Geschwindigkeit größer ist, und der Geschwindigkeit, die so groß ist, dass nur auf 5% der Querschnittsfläche die Geschwindigkeit kleiner ist, gebildet. Je kleiner diese Differenz ist, umso gleichmäßiger ist die Strömungsverteilung.

Figur 11 zeigt Häufigkeitsfunktionen der relativen Geschwindigkeit am Eintritt 11 in die trennwirksamen Einbauten 9, also in die Böden, für die unterschiedlichen Stutzenanordnungen. Hierbei ist die Geschwindigkeit in m/s auf der Abszisse 31 und der kumulierte Flächenanteil von 0 (gar kein Anteil) bis 1 (die gesamte Fläche) auf der Ordinate 33 aufgetragen.

In einer ersten Anordnung beträgt der Winkel α zwischen den Stutzen 3, 5 60°. Die zugehörige vierte Kurve (Strichpunktlinie) der Häufigkeitsfunktion ist mit Bezugszeichen 44 bezeichnet. Eine zweite Kurve 45 (durchgezogene Line) zeigt die Häufigkeitsfunktion für eine Anordnung der Stutzen 3, 5 mit einem Winkel α von 90° zueinander, eine dritte Kurve 46 (gepunktete Linie) die Häufigkeitsfunktion für eine Anordnung der Stutzen 3, 5 mit einem Winkel α von 120°und eine vierte Kurve 47 (gestrichelte Linie) die Häufigkeitsfunktion für eine Anordnung der Stutzen 3, 5 mit einem Winkel α von 180°.

Der Schnittpunkt der Kurven 44, 45, 46 und 47 mit einem kumulierten Flächenanteil 41 von 0,95 bezeichnet die Geschwindigkeit, die so groß ist, dass nur auf 5 von 100 Anteilen der Querschnittsfläche die Geschwindigkeit größer ist und der Schnittpunkt der Kurven 44, 45, 46 und 47 mit dem kumulierten Flächenanteil 43 von 0,05 bezeichnet die Geschwindigkeit, die so groß ist, dass nur auf 5 von 100 Anteilen der Querschnittsfläche die Geschwindigkeit kleiner ist. Die Differenz kann dann auf einfache Weise aus den Graphen bestimmt werden. Wenn alle Kurven 44, 45, 46 und 47 wie hier dargestellt in einem Diagramm gezeigt sind, kann die Ungleichverteilung unmittelbar abgelesen werden. Je größer der Abstand zwischen den Schnittpunkten jeweils einer der Kurven 44, 45, 46 und 47 mit der Gerade 41 beziehungsweise 43 ist, umso größer ist die Ungleichverteilung. Bei einer Anordnung von zwei Stutzen 3, 5, ergibt sich damit, dass hier die größte Ungleichverteilung bei dem Winkel α der Stutzen von 180° vorliegt. Die Ergebnisse, die in Figur 11 dargestellt sind, sind in Tabelle 1 zusammengefasst.

**Tabelle 1**

| Winkel α | Ungleichverteilung bei einem Druckverlust von 3 mbar [%] |
|---|---|
| 60° | 19,3 |
| 90° | 18,6 |
| 120° | 17,9 |
| 180° | 20,0 |

### Beispiel 4

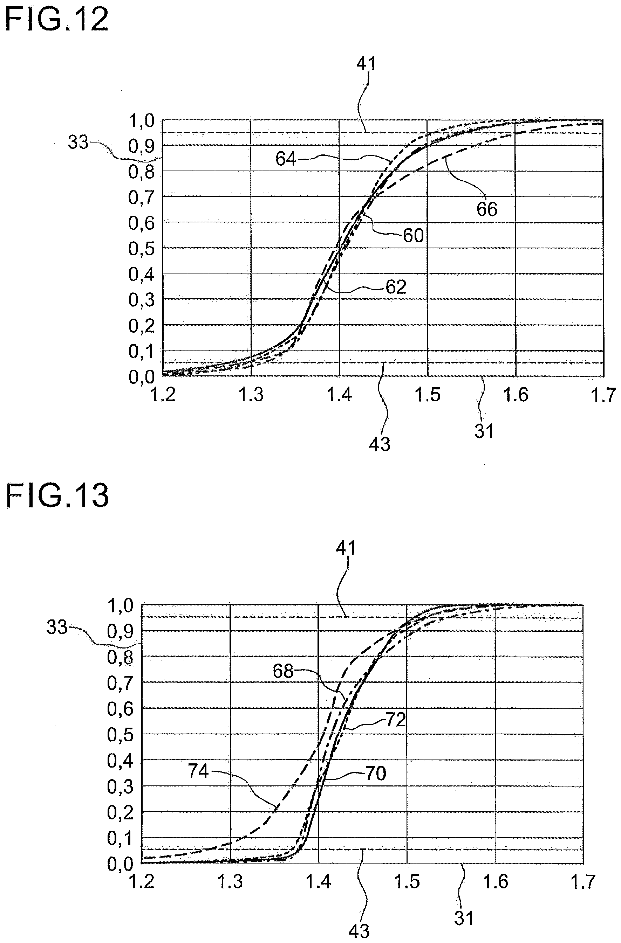

In Figur 12 werden Häufigkeitsverteilungen gezeigt für eine Kolonne 2, die im Wesentlichen der Kolonne 2 gemäß Beispiel 3 entspricht. Hier liegen in den zwei Stutzen 3, 5 unterschiedliche Geschwindigkeiten vor, wobei die Stutzendurchmesser 6, 17 jeweils 900 mm betragen.

Für die Berechnung wurde im ersten Stutzen 3 eine Geschwindigkeit von 8,9 m/s mit einem F-Faktor von 11,3 und im zweiten Stutzen 5 eine Geschwindigkeit von 5,9 m/s mit einem F-Faktor von 7,5 vorgegeben.

Figur 12 zeigt entsprechend Figur 11 Häufigkeitsfunktionen der relativen Geschwindigkeit am Eintritt 11 in die trennwirksamen Einbauten 9, für unterschiedliche Stutzenanordnungen, wobei hier unterschiedliche Gasgeschwindigkeiten in den Stutzen 3, 5 vorliegen.

In einer ersten Anordnung beträgt der Winkel α zwischen den Stutzen 3, 5 60°. Die zugehörige achte Kurve (Strichpunktlinie) der Häufigkeitsfunktion ist mit Bezugszeichen 60 bezeichnet. Eine neunte Kurve 62 (durchgezogene Line) zeigt die Häufigkeitsfunktion für eine Anordnung der Stutzen 3, 5 mit einem Winkel α von 90° zueinander, eine zehnte Kurve 64 (gepunktete Linie) die Häufigkeitsfunktion für eine Anordnung der Stutzen 3, 5 mit einem Winkel α von 120°und eine elfte Kurve 66 (gestrichelte Linie) die Häufigkeitsfunktion für eine Anordnung der Stutzen 3, 5 mit einem Winkel α von 180°.

Die größte Ungleichverteilung liegt bei dem Winkel α der Stutzen von 180° vor, wobei die Gleichverteilung für die verbleibenden Anordnungen gegenüber einer Ausführung mit gleichen Geschwindigkeiten (vgl. Tabelle 1) weiter verbessert wird. Die Ergebnisse, die in Figur 12 dargestellt sind, sind in Tabelle 2 zusammengefasst.

**Tabelle 2**

| Winkel α | Ungleichverteilung bei einem Druckverlust von 3 mbar [%] |
|---|---|
| 60° | 15,6 |
| 90° | 18,6 |
| 120° | 15,1 |
| 180° | 22,0 |

### Beispiel 5

In Figur 13 werden Häufigkeitsverteilungen gezeigt für eine Kolonne 2, die im Wesentlichen der Kolonne 2 gemäß Beispiel 3 entspricht. Hier liegen zwei Stutzen 3, 5 mit unterschiedlichen Stutzendurchmessern 6, 17 vor, wobei die Gasgeschwindigkeiten in den Stutzen 3, 5 jeweils 7,4 m/s mit einem F-Faktor von 9,4 betragen.

Für die Berechnung wurde ein Stutzendurchmesser 6 des ersten Stutzens 3 von 794 mm und ein weiterer Stutzendurchmesser 17 des zweiten Stutzens 5 von 995 mm vorgegeben.

Figur 13 zeigt entsprechend Figur 11 Häufigkeitsfunktionen der relativen Geschwindigkeit am Eintritt 11 in die trennwirksamen Einbauten 9, für unterschiedliche Stutzenanordnungen, wobei hier unterschiedliche Stutzendurchmesser 6, 17 der Stutzen 3, 5 vorliegen.

In einer ersten Anordnung beträgt der Winkel α zwischen den Stutzen 3, 5 60°. Die zugehörige zwölfte Kurve (Strichpunktlinie) der Häufigkeitsfunktion ist mit Bezugszeichen 68 bezeichnet. Eine dreizehnte Kurve 70 (durchgezogene Line) zeigt die Häufigkeitsfunktion für eine Anordnung der Stutzen 3, 5 mit einem Winkel α von 90° zueinander, eine vierzehnte Kurve 72 (gepunktete Linie) die Häufigkeitsfunktion für eine Anordnung der Stutzen 3, 5 mit einem Winkel α von 120°und eine fünfzehnte Kurve 74 (gestrichelte Linie) die Häufigkeitsfunktion für eine Anordnung der Stutzen 3, 5 mit einem Winkel α von 180°.

Die größte Ungleichverteilung liegt bei dem Winkel α der Stutzen von 180° vor, wobei die Gleichverteilung gegenüber einer Ausführung mit gleichen Stutzendurchmessern (vgl. Tabelle 1) weiter verbessert wird. Die Ergebnisse, die in Figur 13 dargestellt sind, sind in Tabelle 3 zusammengefasst.

**Tabelle 3**

| Winkel α | Ungleichverteilung bei einem Druckverlust von 3 mbar [%] |
|---|---|
| 60° | 12,1 |
| 90° | 9,3 |
| 120° | 13,7 |
| 180° | 18,2 |

### Beispiel 6

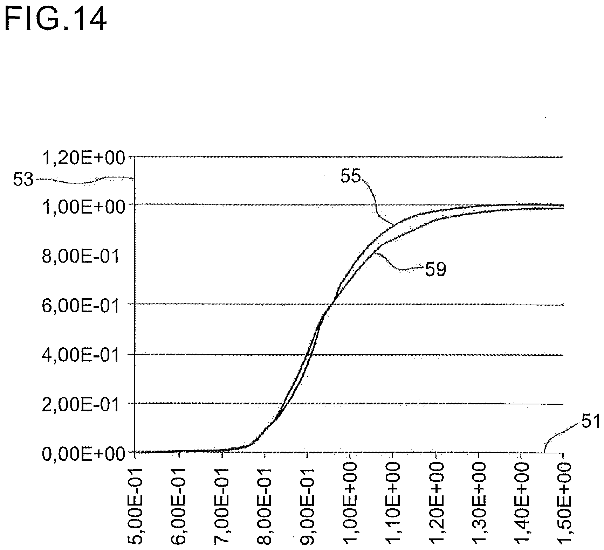

Als Randbedingungen bezüglich Figur 14 wurden die gleichen gewählt wie für die Darstellung in Figur 9 mit dem Unterschied, dass die Geschwindigkeit in den Stutzen 3, 5 um den Faktor 1,5 erhöht worden ist und um die gleiche Geschwindigkeit in der Kolonne zu erhalten die Stutzendurchmesser 6, 17 entsprechend verkleinert wurden.

Bei einer Kurve 55, die die Häufigkeitsverteilung für zwei Stutzen 3, 5 zeigt, wurde ein Winkel α zwischen den Stutzen 3, 5 von 120° vorgegeben. Für die Berechnung einer Kurve 59 mit Verwendung von nur einem Stutzen 3 wurde ein um Faktor 2^{0,5} im Durchmesser größerer Stutzen 3 eingesetzt. Hierdurch bleiben der F-Faktor und die Geschwindigkeit in der Kolonne 2 und damit auch der Druckverlust in den trennwirksamen Einbauten 9, also der Packung, im Vergleich zur Zufuhr der dampfförmigen Phase über zwei Stutzen 3, 5 konstant.

Auf der Abszisse 51 ist hier die normierte Geschwindigkeit und auf der Ordinate 53 der Flächenanteil aufgetragen.

Die erste Kurve 55 zeigt die Häufigkeitsverteilung für zwei Stutzen 3, 5. Die zweite Kurve 59 zeigt die Häufigkeitsverteilung bei einem Stutzen 3.

Aus dem Vergleich in Figur 14 lässt sich klar entnehmen, dass bei einer Kolonne 2 mit zwei Stutzen 3, 5 eine bessere Gleichverteilung erzielt wird.

### Beispiel 7

Anhand einer thermodynamischen Simulation einer Gesamtanlage zur Herstellung von Acrylsäure-n-butylester wurden die Auswirkungen einer Ungleichverteilung einer aus zwei Verdampfern austretenden Dampfphase, bezogen auf die Querschnittsfläche einer Kolonne, untersucht.

Die thermodynamische Simulation wurde mit der Software Aspen Plus^{®} durchgeführt. Modell-Datenbanken zur Modellierung von Basisoperationen sowie Stoffdatenbanken bezüglich spezifischer Stoffeigenschaften, die in der Software implementiert sind, wurden eingebracht. Mischungseigenschaften wurden mittels der Software basierend auf verschiedenen thermodynamischen Modellen aus Stoffdaten der reinen Substanzen berechnet.

### Beispiel 7a

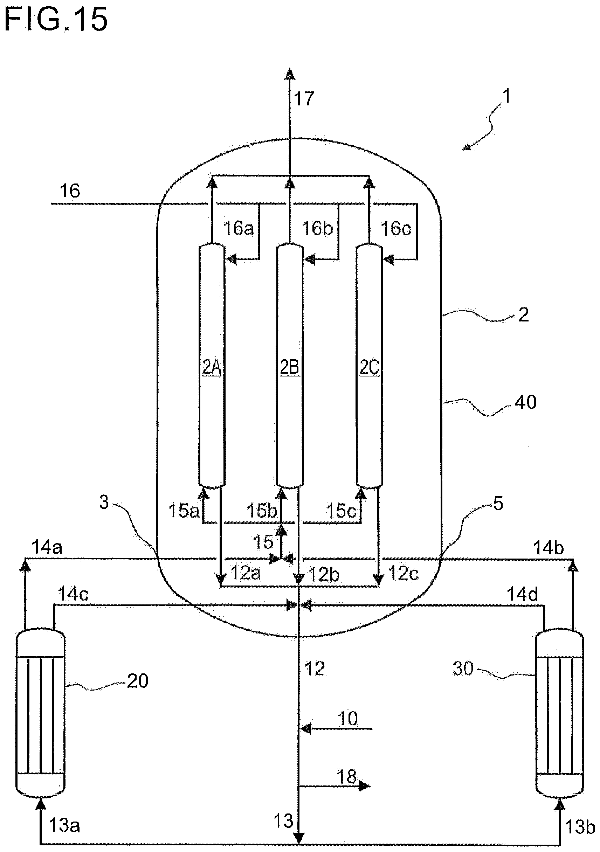

Zur Ermittlung des Energiebedarfs bei einer gleichverteilten dampfförmigen Phase wurden drei gleiche Dampfmengenströme 15a, 15b, 15c, die in Figur 15 dargestellt sind, zugrunde gelegt.

Eine Kolonne 2 als Rektifikationskolonne 40, wurde mit drei gleichen Teilkolonnen 2A, 2B, 2C in der Simulation dargestellt und die drei Teilkolonnen 2A, 2B, 2C wurden jeweils mit 13 theoretischen Trennstufen simuliert.

Am Kopf der Teilkolonnen 2A, 2B, 2C wurde ein Rücklauf 16 in Form einer flüssigen Phase in drei gleich große Flüssigkeitsmengenströme 16a, 16b, 16c aufgeteilt und auf die drei Teilkolonnen 2A, 2B, 2C aufgegeben.

Am Kolonnensumpf wurden Sumpfabzüge 12a, 12b, 12c und aus Verdampfern 20, 30 austretende Flüssigkeitsströme 14c, 14d zu einem Gesamtstrom 12, also einer flüssigen Phase der Kolonne 2, zusammengefasst und mit einem Zulaufstrom 10 gemischt. Ein kleiner Teilstrom 18 des Gesamtstroms 12 wurde aus der Anlage herausgeführt und der Hauptstrom 13 des Gesamtstroms 12 wurde auf zwei gleich große Mengenströme 13a, 13b aufgeteilt und den beiden Verdampfern 20, 30 zugeführt.

Aus den Verdampfern 20, 30 austretende Dampfströme 14a, 14b, die beispielsweise durch Stutzen 3, 5 der Kolonne 2 zugeführt wurden, wurden zusammengeführt zu einem Dampfzufuhrstrom 15 und dann in drei gleich große Mengenströme 15a, 15b, 15c, jeweils in Form einer dampfförmigen Phase, aufgeteilt und in die drei Teilkolonnen 2A, 2B, 2C eingeleitet.

Die Aufteilung des Dampfzufuhrstromes 15 erfolgte gleichmäßig:

| | |
|---|---|
| Dampfstrom (15a) | 33,33 Gew.-%, |
| Dampfstrom (15b) | 33,33 Gew.-%, |
| Dampfstrom (15c) | 33,33 Gew.-%, jeweils bezogen auf den Dampfzufuhrstrom 15 |

Die thermodynamische Simulation der Gesamtanlage ergab folgende benötigte Wärmemengen in den Verdampfern 20, 30:

| | |
|---|---|
| Verdampfer 20: | 8922 kW, |
| Verdampfer 30: | 8922 kW. |

### Beispiel 7b

Zur Ermittlung des Energiebedarfs bei einer ungleichverteilten dampfförmigen Phase wurden drei ungleiche Dampfmengenströme 15a, 15b, 15c gemäß Figur 15 zugrunde gelegt. Ansonsten wurde wie in Beispiel 7a verfahren.

Durch die unterschiedlichen Dampfmengenströme 15a, 15b, 15c, die zu den Teilkolonnen 2A, 2B, 2C geleitet wurden, wurde eine Ungleichverteilung der aus den Verdampfern 20, 30 austretenden Dampfströme 14a, 14b auf die Querschnittsfläche der Kolonne 2, die durch eine ungünstige Anordnung der Stutzen 3, 5 am Kolonnenumfang bedingt ist, simuliert.

Die Aufteilung des Dampfzufuhrstromes 15 erfolgte ungleichmäßig:

| | |
|---|---|
| Dampfstrom (15a) | 31,33 Gew.-%, |
| Dampfstrom (15b) | 33,33 Gew.-%, |
| Dampfstrom (15c) | 35,33 Gew.-%, jeweils bezogen auf den Dampfzufuhrstrom 15 |

Alle anderen Bedingungen blieben im Vergleich zu Beispiel 7a unverändert.

Die thermodynamische Simulation der Gesamtanlage ergab folgende benötigte Wärmemengen in den Verdampfern 20, 30:

| | |
|---|---|
| Verdampfer (20): | 9237 kW, |
| Verdampfer (30): | 9237 kW. |

Im Vergleich zu Beispiel 7a wurde mit der ungleichmäßigen Dampfzufuhr etwa 3,5 % mehr Energie in den beiden Verdampfern benötigt.

Durch die Ungleichverteilung der zugeführten dampfförmigen Phase wurden lokale Verhältnisse von flüssiger Phase und dampfförmiger Phase in der Rektifikationskolonne 40 verändert. Um die gleiche Trennaufgabe in der Rektifikationskolonne 40 wie in Beispiel 7a zu erfüllen, wird mehr Energie als bei einer Gleichverteilung der dampfförmigen Phase benötigt.

Ein Vergleich der Beispiele 7a und 7b unter Berücksichtigung der Beispiele 1 bis 6 zeigt, dass durch eine Gleichverteilung der dampfförmigen Phase auf die Querschnittsfläche einer Kolonne 2, insbesondere einer Rektifikationskolonne 40, die durch eine erfindungsmäße Ausgestaltung der Stutzen 3, 5 erzielt wird, der Energiebedarf für die gleiche Trennleistung verringert wird.

### Bezugszeichenliste

- 1: Vorrichtung zur Durchführung von Stoffaustauschprozessen
- 2: Kolonne
- 2A, 2B, 2C: Teilkolonnen
- 3: erster Stutzen
- 4: radiale Richtung
- 5: zweiter Stutzen
- 6: Stutzendurchmesser
- 7: Kolonnendurchmesser
- 8: Höhe
- 9: trennwirksame Einbauten
- 10: Zulaufstrom
- 11: Eintritt
- 12: Gesamtstrom, flüssige Phase
- 12a, 12b, 12c: Sumpfabzüge
- 13: Hauptstrom
- 13a, 13b: Mengenströme des Hauptstroms
- 14a, 14b: austretende Dampfströme
- 14c, 14d: austretende Flüssigkeitsströme
- 15: Dampfzufuhrstrom
- 15a, 15b, 15c: Dampfmengenströme des Dampfzufuhrstroms
- 16: Rücklauf16a, 16b, 16c Flüssigkeitsmengenströme
- 17: weiterer Stutzendurchmesser
- 18: Teilstrom
- 19: Umfang
- 20: erster Verdampfer
- 21: Abszisse, Geschwindigkeit
- 23: Ordinate, Querschnittsfläche
- 25: dritter Stutzen
- 27: vierter Stutzen
- 28: Abschnittshöhe
- 29: Kolonnenabschnitt
- 30: zweiter Verdampfer
- 31: Abszisse, relative Geschwindigkeit
- 33: Ordinate, kumulierter Flächenanteil
- 35: erste Kurve der Häufigkeitsfunktion
- 37: zweite Kurve der Häufigkeitsfunktion
- 39: dritte Kurve der Häufigkeitsfunktion
- 40: Rektifikationskolonne
- 41: kumulierter Flächenanteil von 95%
- 43: kumulierter Flächenanteil von 5%
- 44: vierte Kurve der Häufigkeitsfunktion
- 45: fünfte Kurve der Häufigkeitsfunktion
- 46: sechste Kurve der Häufigkeitsfunktion
- 47: siebte Kurve der Häufigkeitsfunktion
- 51: Abszisse, normierte Geschwindigkeit
- 53: Ordinate, Flächenanteil
- 55: erste Kurve der Häufigkeitsverteilung
- 59: zweite Kurve der Häufigkeitsverteilung
- 60: achte Kurve der Häufigkeitsfunktion
- 62: neunte Kurve der Häufigkeitsfunktion
- 64: zehnte Kurve der Häufigkeitsfunktion
- 66: elfte Kurve der Häufigkeitsfunktion
- 68: zwölfte Kurve der Häufigkeitsfunktion
- 70: dreizehnte Kurve der Häufigkeitsfunktion
- 72: vierzehnte Kurve der Häufigkeitsfunktion
- 74: fünfzehnte Kurve der Häufigkeitsfunktion
- α: Winkel
- β: weiterer Winkel

## Patentansprüche

1. Vorrichtung (1) zur Durchführung von Stoffaustauschprozessen, umfassend eine Kolonne (2) mit mindestens zwei Stutzen (3, 5) zur Zufuhr einer dampfförmigen Phase,
wobei in der Kolonne (2) trennwirksame Einbauten (9) aufgenommen sind und sich ein Kolonnenabschnitt (29) von den mindestens zwei Stutzen (3, 5) zu den trennwirksamen Einbauten (9) erstreckt, in dem eine Bedeckung einer Querschnittsfläche der Kolonne (2) weniger als 25% beträgt, bezogen auf die gesamte Querschnittsfläche, und
wobei die mindestens zwei Stutzen (3, 5) einen Höhenversatz aufweisen, der maximal dem Dreifachen des größten Stutzendurchmessers (6) entspricht, und die mindestens zwei Stutzen (3, 5) einen Winkel (α) von 60° bis 150° zueinander aufweisen und eine Asymmetrie zueinander aufweisen, wobei die Asymmetrie dadurch gegeben ist, dass die mindestens zwei Stutzen (3, 5) jeweils unterschiedliche Stutzendurchmesser (6, 17) aufweisen.

2. Vorrichtung (1) gemäß Anspruch 1, **dadurch gekennzeichnet, dass** die Asymmetrie zudem dadurch gegeben ist, dass die mindestens zwei Stutzen (3, 5) asymmetrisch über den Umfang (19) der Kolonne (2) verteilt sind.

3. Vorrichtung (1) gemäß Anspruch 1 oder 2, **dadurch gekennzeichnet, dass** sich der Winkel (α) um mindestens 10°, insbesondere mindestens 30°, von einem weiteren Winkel (β) zwischen zwei der mindestens zwei Stutzen (3, 5) unterscheidet.

4. Vorrichtung (1) gemäß einem der Ansprüche 1 bis 3, **dadurch gekennzeichnet, dass** die mindestens zwei Stutzen (3, 5) in gleicher Höhe (8) an der Kolonne (2) angeordnet sind.

5. Vorrichtung (1) gemäß einem der Ansprüche 1 bis 4, **dadurch gekennzeichnet, dass** die mindestens zwei Stutzen (3, 5) am Sumpf der Kolonne (2) oder als Seitenzulauf an der Kolonne (2) angeordnet sind.

6. Vorrichtung gemäß einem der Ansprüche 1 bis 5, **dadurch gekennzeichnet, dass** die Vorrichtung (1) genau zwei Stutzen (3, 5) zur Zufuhr einer dampfförmigen Phase umfasst, wobei die zwei Stutzen (3, 5) einen Höhenversatz aufweisen, der maximal dem Dreifachen des größten Stutzendurchmessers (6) entspricht.

7. Vorrichtung (1) gemäß einem der Ansprüche 1 bis 6, **dadurch gekennzeichnet, dass** die mindestens zwei Stutzen (3, 5) radial in die Kolonne (2) münden.

8. Vorrichtung (1) gemäß einem der Ansprüche 1 bis 7, **dadurch gekennzeichnet, dass** die trennwirksamen Einbauten (9) eine strukturierte Packung und/oder Füllkörper umfassen.

9. Vorrichtung (1) gemäß einem der Ansprüche 1 bis 8, **dadurch gekennzeichnet, dass** die trennwirksamen Einbauten (9) Böden ohne Zwangsführung wie Dual-Flow-Böden, Ripple-Tray-Böden und/oder Kaskadenböden umfassen.

10. Vorrichtung (1) gemäß einem der Ansprüche 1 bis 8, **dadurch gekennzeichnet, dass** die trennwirksamen Einbauten (9) Querstromböden umfassen.

11. Vorrichtung (1) gemäß einem der Ansprüche 1 bis 10, **dadurch gekennzeichnet, dass** über die mindestens zwei Stutzen (3, 5) Verdampfer (20, 30) an die Kolonne (2) angebunden sind.

12. Verwendung der Vorrichtung (1) zur Durchführung von Stoffaustauschprozessen gemäß einem der Ansprüche 1 bis 11 zur Herstellung von Isocyanaten, Styrol oder eines Acrylsäurealkylesters, insbesondere eines Acrylsäurebutylesters, oder in Crackern, insbesondere zum Splitten von C₃-Kohlenwasserstoffen.

13. Verwendung gemäß Anspruch 12, **dadurch gekennzeichnet, dass** die Asymmetrie dadurch gegeben ist, dass die mindestens zwei Stutzen (3, 5) mit jeweils unterschiedlichen mittleren Geschwindigkeiten durchströmt werden.

14. Verwendung gemäß Anspruch 12 oder 13, **dadurch gekennzeichnet, dass** die Vorrichtung (1) zur Durchführung von Stoffaustauschprozessen als Rektifikationskolonne (40) verwendet wird in einem Verfahren zur kontinuierlichen Herstellung eines Acrylsäurealkylesters (H₂C=CH-C(=O)OR, mit R = n-Butyl oder iso-Butyl), wobei wässrige 3-Hydroxypropionsäure unter dehydratisierenden und veresternden Bedingungen in Gegenwart des entsprechenden Butanols (R-OH) in einem Reaktor mit der Rektifikationskolonne (40) umgesetzt wird und gebildeter Acrylsäurebutylester, unumgesetztes Butanol sowie eingesetztes und gebildetes Wasser als ternäres Azeotrop über Kopf abdestilliert wird und nach Trennung in eine jeweils flüssige wässrige und organische Phase die wässrige und die organische Phase jeweils zumindest teilweise ausgeschleust und die organische Phase, enthaltend den Acrylsäurebutylester und das Butanol, destillativ aufgetrennt wird.

15. Verwendung gemäß einem der Ansprüche 12 bis 14, **dadurch gekennzeichnet, dass** eine flüssige Phase (12) der Rektifikationskolonne (40) entnommen, zumindest teilweise verdampft und zumindest teilweise über die mindestens zwei Stutzen (3, 5) in die Rektifikationskolonne (40) zurückgeführt wird.

16. Verwendung gemäß Anspruch 15, **dadurch gekennzeichnet, dass** die flüssige Phase (12) in mindestens zwei Verdampfern (20, 30) zumindest teilweise verdampft wird.

17. Verwendung gemäß einem der Ansprüche 12 bis 16, **dadurch gekennzeichnet, dass** der Druck am Kopf der Rektifikationskolonne (40) im Bereich von 0,2 bar bis 5,0 bar absolut liegt.

18. Verwendung gemäß einem der Ansprüche 14 bis 17, **dadurch gekennzeichnet, dass** die destillative Auftrennung der organischen Phase, enthaltend den Acrylsäurebutylester und das Butanol, derart erfolgt, dass in einer zusätzlichen Rektifikationskolonne (40) das Butanol abdestilliert und aus der resultierenden Sumpfflüssigkeit in einer weiteren zusätzlichen Rektifikationskolonne (40) der Acrylsäurebutylester abdestilliert wird.

19. Verfahren zur Auslegung der Vorrichtung (1) zum Stoffaustausch gemäß einem der Ansprüche 1 bis 11 folgende Schritte umfassend:
(a) Vorgabe der Position und Ausrichtung der mindestens zwei Stutzen (3, 5) an der Kolonne (2);
(b) Berechnen der Gasströmung in der Kolonne (2) mit einer Strömungssimulation;
(c) Wiederholen der Schritte (a) und (b) mit unterschiedlichen Positionen und Ausrichtungen der mindestens zwei Stutzen (3, 5) und
(d) Auswahl der Position und Ausrichtung der mindestens zwei Stutzen (3, 5) bei der Strömung, die den gleichmäßigsten Strömungsverlauf zeigt.

## Claims

1. An apparatus (1) for carrying out mass transfer processes, comprising a column (2) having at least two inlet pipes (3, 5) for introducing a gaseous phase,
where separation-active internals (9) are accommodated in the column (2) and a column section (29) extends from the at least two inlet pipes (3, 5) to the separation-active internals (9), in which section a coverage of a cross-sectional area of the column (2) is less than 25%, based on the total cross-sectional area, and
where the at least two inlet pipes (3, 5) have a height offset which corresponds to not more than three times the largest inlet pipe diameter (6) and the at least two inlet pipes (3, 5) are at an angle (α) of from 60° to 150° to one another and have asymmetry with respect to one another, wherein the asymmetry is given by the at least two inlet pipes (3, 5) each having different inlet pipe diameters (6, 17).

2. The apparatus (1) according to claim 1, wherein the asymmetry is additionally given by the at least two inlet pipes (3, 5) being distributed asymmetrically around the circumference (19) of the column (2).

3. The apparatus (1) according to claim 1 or 2, wherein the angle (α) differs by at least 10°, in particular at least 30°, from a further angle (β) between two of the at least two inlet pipes (3, 5).

4. The apparatus (1) according to any of claims 1 to 3, wherein the at least two inlet pipes (3, 5) are arranged at the same height (8) on the column (2).

5. The apparatus (1) according to any of claims 1 to 4, wherein the at least two inlet pipes (3, 5) are arranged at the bottom of the column (2) or as side inlet on the column (2).

6. The apparatus according to any of claims 1 to 5, wherein the apparatus (1) comprises precisely two inlet pipes (3, 5) for introducing a gaseous phase, where the two inlet pipes (3, 5) have a height offset which corresponds to not more than three times the largest inlet pipe diameter (6).

7. The apparatus (1) according to any of claims 1 to 6, wherein the at least two inlet pipes (3, 5) open radially into the column (2).

8. The apparatus (1) according to any of claims 1 to 7, wherein the separation-active internals (9) comprise a structured packing and/or packing elements.

9. The apparatus (1) according to any of claims 1 to 8, wherein the separation-active internals (9) comprise trays without guided flow, for example dual-flow trays, ripple trays and/or cascade trays.

10. The apparatus (1) according to any of claims 1 to 8, wherein the separation-active internals (9) comprise crossflow trays.

11. The apparatus (1) according to any of claims 1 to 10, wherein vaporizers (20, 30) are attached via the at least two inlet pipes (3, 5) to the column (2).

12. The use of the apparatus (1) for carrying out mass transfer processes according to any of claims 1 to 11 for producing isocyanates, styrene or an alkyl acrylate, in particular a butyl acrylate, or in crackers, in particular for dissociating C₃-hydrocarbons.

13. The use according to claim 12, wherein the asymmetry is given by the average flow velocities through the at least two inlet pipes (3, 5) being different.

14. The use according to claim 12 or 13, wherein the apparatus (1) for carrying out mass transfer processes is used as rectification column (40) in a process for the continuous production of an alkyl acrylate (H₂C=CH-C(=0)OR, where R = n-butyl or isobutyl), where aqueous 3-hydroxypropionic acid is reacted under dehydrating and esterifying conditions in the presence of the appropriate butanol (R-OH) in a reactor comprising the rectification column (40) and butyl acrylate formed, unreacted butanol and also water used and water formed are distilled off as ternary azeotrope at the top and, after separation into a respectively liquid aqueous phase and liquid organic phase, the aqueous phase and the organic phase are each at least partly discharged and the organic phase comprising the butyl acrylate and the butanol is fractionally distilled.

15. The use according to any of claims 12 to 14, wherein a liquid phase (12) is taken off from the rectification column (40), at least partially vaporized and at least partly recirculated via the at least two inlet pipes (3, 5) to the rectification column (40).

16. The use according to claim 15, wherein the liquid phase (12) is at least partially vaporized in at least two vaporizers (20, 30).

17. The use according to any of claims 12 to 16, wherein the pressure at the top of the rectification column (40) is in the range from 0.2 bar to 5.0 bar absolute.

18. The use according to any of claims 14 to 17, wherein the fractional distillation of the organic phase comprising the butyl acrylate and the butanol is carried out by distilling off the butanol in an additional rectification column (40) and distilling off the butyl acrylate from the resulting bottoms in a further additional rectification column (40).

19. A method for designing the apparatus (1) for mass transfer according to any of claims 1 to 11, comprising the following steps:
(a) specification of the position and orientation of the at least two inlet pipes (3, 5) on the column (2);
(b) calculation of the gas flow in the column (2) using a flow simulation;
(c) repetition of the steps (a) and (b) with different positions and orientations of the at least two inlet pipes (3, 5) and
(d) selection of the position and orientation of the at least two inlet pipes (3, 5) at the flow which displays the most uniform flow pattern.

## Revendications

1. Dispositif (1) pour la réalisation de processus d'échange de matière, comprenant une colonne (2) avec au moins deux tubulures (3, 5) pour l'amenée d'une phase sous forme de vapeur, des éléments internes à action de séparation (9) étant reçus dans la colonne (2) et une section de colonne (29) s'étendant des au moins deux tubulures (3, 5) aux éléments internes à action de séparation (9), dans laquelle un recouvrement d'une surface de section transversale de la colonne (2) est inférieur à 25 %, par rapport à la surface de section transversale totale, et les au moins deux tubulures (3, 5) présentant un décalage en hauteur qui correspond au maximum au triple du plus grand diamètre de tubulure (6), et les au moins deux tubulures (3, 5) présentant un angle (α) de 60° à 150° l'une par rapport à l'autre et présentant une asymétrie l'une par rapport à l'autre, l'asymétrie résultant du fait que les au moins deux tubulures (3, 5) présentent chacune des diamètres de tubulure différents (6, 17).

2. Dispositif (1) selon la revendication 1, **caractérisé en ce que** l'asymétrie résulte en outre du fait que les au moins deux tubulures (3, 5) sont réparties de manière asymétrique sur la circonférence (19) de la colonne (2).

3. Dispositif (1) selon la revendication 1 ou 2, **caractérisé en ce que** l'angle (α) diffère d'au moins 10°, notamment d'au moins 30°, d'un autre angle (β) entre deux des au moins deux tubulures (3, 5).

4. Dispositif (1) selon l'une quelconque des revendications 1 à 3, **caractérisé en ce que** les au moins deux tubulures (3, 5) sont agencées à la même hauteur (8) sur la colonne (2).

5. Dispositif (1) selon l'une quelconque des revendications 1 à 4, **caractérisé en ce que** les au moins deux tubulures (3, 5) sont agencées au fond de la colonne (2) ou en tant qu'entrée latérale de la colonne (2).

6. Dispositif selon l'une quelconque des revendications 1 à 5, **caractérisé en ce que** le dispositif (1) comprend exactement deux tubulures (3, 5) pour l'amenée d'une phase sous forme vapeur, les deux tubulures (3, 5) présentant un décalage en hauteur qui correspond au maximum à trois fois le plus grand diamètre de tubulure (6).

7. Dispositif (1) selon l'une quelconque des revendications 1 à 6, **caractérisé en ce que** les au moins deux tubulures (3, 5) débouchent radialement dans la colonne (2).

8. Dispositif (1) selon l'une quelconque des revendications 1 à 7, **caractérisé en ce que** les éléments internes à action de séparation (9) comprennent un garnissage structuré et/ou des corps de remplissage.

9. Dispositif (1) selon l'une quelconque des revendications 1 à 8, **caractérisé en ce que** les éléments internes à action de séparation (9) comprennent des plateaux sans guidage forcé tels que des plateaux Dual Flow, des plateaux Ripple Tray et/ou des plateaux à cascade.

10. Dispositif (1) selon l'une quelconque des revendications 1 à 8, **caractérisé en ce que** les éléments internes à action de séparation (9) comprennent des plateaux à flux transversal.

11. Dispositif (1) selon l'une quelconque des revendications 1 à 10, **caractérisé en ce que** des évaporateurs (20, 30) sont reliés à la colonne (2) par l'intermédiaire des au moins deux tubulures (3, 5).

12. Utilisation du dispositif (1) pour la réalisation de processus d'échange de matière selon l'une quelconque des revendications 1 à 11 pour la production d'isocyanates, de styrène ou d'un ester alkylique d'acide acrylique, notamment d'un ester butylique d'acide acrylique, ou dans des craqueurs, notamment pour le fractionnement d'hydrocarbures en C₃.

13. Utilisation selon la revendication 12, **caractérisée en ce que** l'asymétrie résulte du fait que les au moins deux tubulures (3, 5) sont traversées par des vitesses moyennes respectivement différentes.

14. Utilisation selon la revendication 12 ou 13, **caractérisée en ce que** le dispositif (1) pour la réalisation de processus d'échange de matière est utilisé en tant que colonne de rectification (40) dans un procédé de production en continu d'un ester alkylique d'acide acrylique (H₂C=CH-C(=O)OR, avec R = n-butyle ou isobutyle), dans lequel de l'acide 3-hydroxypropionique aqueux est mis en réaction dans des conditions de déshydratation et d'estérification en présence du butanol correspondant (R-OH) dans un réacteur avec la colonne de rectification (40) et l'ester butylique d'acide acrylique formé, le butanol n'ayant pas réagi ainsi que l'eau utilisée et formée sont distillés par la tête sous forme d'azéotrope ternaire et, après séparation en une phase aqueuse et une phase organique respectivement liquides, la phase aqueuse et la phase organique sont chacune au moins partiellement évacuées et la phase organique, contenant l'ester butylique d'acide acrylique et le butanol, est séparée par distillation.

15. Utilisation selon l'une quelconque des revendications 12 à 14, **caractérisée en ce qu'**une phase liquide (12) est prélevée de la colonne de rectification (40), au moins partiellement évaporée et au moins partiellement recyclée dans la colonne de rectification (40) par l'intermédiaire des au moins deux tubulures (3, 5) .

16. Utilisation selon la revendication 15, **caractérisée en ce que** la phase liquide (12) est au moins partiellement évaporée dans au moins deux évaporateurs (20, 30).

17. Utilisation selon l'une quelconque des revendications 12 à 16, **caractérisée en ce que** la pression à la tête de la colonne de rectification (40) se situe dans la plage de 0,2 bar à 5,0 bars absolus.

18. Utilisation selon l'une quelconque des revendications 14 à 17, **caractérisée en ce que** la séparation par distillation de la phase organique contenant l'ester butylique d'acide acrylique et le butanol est effectuée de telle sorte que le butanol est distillé dans une colonne de rectification supplémentaire (40) et l'ester butylique d'acide acrylique est distillé à partir du liquide de fond résultant dans une autre colonne de rectification supplémentaire (40).

19. Procédé de conception du dispositif (1) pour l'échange de matière selon l'une quelconque des revendications 1 à 11, comprenant les étapes suivantes :
(a) la prédétermination de la position et de l'orientation des au moins deux tubulures (3, 5) sur la colonne (2) ;
(b) le calcul de l'écoulement de gaz dans la colonne (2) avec une simulation d'écoulement ;
(c) la répétition des étapes (a) et (b) avec différentes positions et orientations des au moins deux tubulures (3, 5) ; et
(d) la sélection de la position et de l'orientation des au moins deux tubulures (3, 5) pour l'écoulement qui présente la courbe d'écoulement la plus régulière.
